# EUROPÄISCHE PATENTANMELDUNG

(11) **EP 0 548 883 A1**
(43) Veröffentlichungstag der Anmeldung: **30.06.1993**
(21) Anmeldenummer: 92121706.3
(22) Anmeldetag: 21.12.1992
(51) Int. Cl.: C07D 213/89, A61K 31/44

(54) **Substituierte Pyridin-N-oxide, Verfahren zu ihrer Herstellung sowie ihre Verwendung als Arzneimittel**

(30) Priorität: 24.12.1991 DE 4142989
(71) Anmelder: HOECHST AKTIENGESELLSCHAFT, D-65926 Frankfurt am Main (DE)
(72) Erfinder: Weidmann, Klaus, Dr., W-6242 Kronberg/Ts. (DE); Bickel, Martin, Dr., W-6350 Bad Homburg (DE); Günzler-Pukall, Volkmar, Dr., W-3550 Marburg (DE); Baringhaus, Karl-Heinz, Dr., W-6237 Liederbach/Ts. (DE)

(57) **Zusammenfassung**

Es werden in den Positionen 2 und 4 oder 5 mit Ester- und/oder Amidgruppen substituierte Pyridin-N-oxide beschreiben und ein Verfahren zu ihrer Herstellung. Die genannten Verbindungen wirken als Inhibitoren der Lysin- und Prolinhydroxylase.

## Beschreibung

Verbindungen, die die Enzyme Proin- und Lysinhydroxylase inhibieren, bewirken eine sehr selektive Hemmung der Kollagenbiosynthese durch Beeinflussung der kollagenspezifischen Hydroxylierungsreaktionen. In deren Verlauf wird Proteingebundenes Prolin oder Lysin durch die Enzyme Proin- bzw. Lysinhydroxylase hydroxyliert. Wird diese Reaktion durch Inhibitoren unterbunden, so entsteht ein nicht funktionsfähiges, unterhydroxyliertes Kollagenmolekül, das von den Zellen nur in geringer Menge in den extrazallulären Raum abgegeben werden kann. Das unterhydroxylierte Kollagen kann außerdem nicht in die Kollagenmatrix eingebaut werden und wird sehr leicht proteolytisch abgebaut. Als Folge dieser Effekte verringert sich insgesamt die Menge des extrazellulär abgelagerten Kollagens.

Inhibitoren der Prolylhydroxylase sind deshalb geeignete Substanzen in der Therapie von Erkrankungen, in denen die Ablagerung von Kollagenen maßgeblich zum Krankheitsbild beiträgt. Hierzu gehören u. a. Fibrosen der Lunge, Leber und Haut (Skleroderma) sowie die Atherosklerose.

Es ist bekannt, daß die Inhibierung der Prolinhydroxylase durch bekannte Inhibitoren wie α,α',-Dipyridyl zu einer Hemmung der Cl_{q}-Biosynthese von Makrophagen führt (W. Müller et al., FEBS Lett. 90(1978), 218; Immunbiology 155 (1978), 47). Dadurch kommt es zu einem Ausfall des klassischen Weges der Komplementaktivierung. Inhibitore der Prolinhydroxylase wirken daher auch als Immunsuppressiva, z. B. bei Immunkomplexkrankheiten.

Es ist bekannt, daß das Enzym Prolinhydroxylase durch Pyridin-2,4- und -2,5-dicarbonsäure effektiv gehemmt wird (K. Majamaa et al., Eur. J. Biochem. 138 (1984) 239-245). Diese Verbindungen sind in der Zellkultur allerdings nur in sehr hohen Konzentrationen als Hemmstoffe wirksam (Tschank, G. et al., Biochem. J. 238 (1987) 625-633).

In der DE-A 34 32 094 werden Pyridin-2,4- und -2,5-dicarbonsäurediester mit 1-6 C-Atomen im Esteralkylteil als Arzneimittel zur Inhibierung der Prolin- und Lysinhydroxylase beschrieben.

Diese niedrig-alkylierten bester haben jedoch den Nachteil, daß sie zu schnell im Organismus zu den Säuren gespalten werden und nicht in genügend hoher Konzentration an ihren Wirkort in der Zelle gelangen und damit für eine eventuelle Verabreichung als Arzneimittel weniger geeignet sind.

Die DE-A 37 03 959, DE-A 37 03 962 und DE-A 37 03 963 beschreiben in allgemeiner Form gemischte Ester/Amide, höher alkylierte Diester und Diamide der Pyridin-2,4- und -2,5-dicarbonsäure, die die Kollagenbiosynthese im Tiermodell wirksam hemmen.

In der deutschen Patentanmeldung P 40 20 570.3 werden 2,4- und 2,5-disubstituierte Pyridin-N-oxide beschrieben.

Es wurde nunmehr gefunden, daß auch substituierte Pyridin-N-oxide der folgenden Formel I sowie die physiologisch verträglichen Salze die Lysin- und Prolinhydroxylase wirksam hemmen.

Die Erfindung betrifft daher substituierte Pyridin-N-oxide der allgemeinen Formel I, 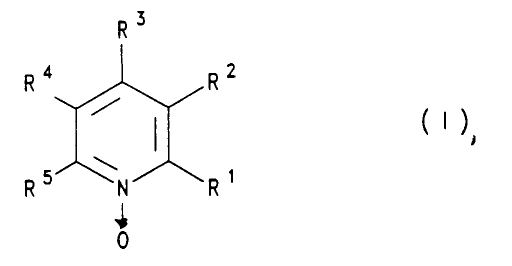
worin
- R¹ und R³ oder R⁴: -C(O)-X-R⁶ bedeuten, wobei
- X: O oder -N (R⁷)- und
- R⁶ und R⁷: gleich oder verschieden sind und
- A: einen verzweigten oder unverzweigten, aliphatischen oder cycloaliphatischen (C₁-C₁₂)-Alkylrest, (C₁-C₁₂)-Alkenylrest oder einen (C₁-C₁₂)-Alkinylrest bedeutet,
der unsubstituiert oder einfach oder mehrfach, vorzugsweise einfach oder zweifach substituiert ist mit Halogen, insbesondere Fluor, Chlor, Brom, Hydroxy, Cyano, Carboxyl, (C₁-C₈)-Alkoxy, (C₁-C₈)-Alkoxycarbonyl, (C₁-C₈)-Alkoxycarbonyloxy, (C₁-C₈)-Alkoxy-(C₁-C₈)-alkoxycarbonyloxy, (C₆-C₁₂)-Aryloxycarbonyloxy, (C₇-C₁₁)-Aralkyloxycarbonyloxy, (C₇-C₁₁)-Aralkylcarbonyloxy, Cinnamoyl, Cinnamoyloxy, (C₆-C₁₂)-Arylcarbonyloxy, (C₃-C₈)-Alkenylcarbonyloxy, (C₃-C₈)-Alkinylcarbonyloxy, (C₃-C₈)-Cycloalkylcarbonyloxy, (C₁-C₁₂)-Alkoxy-(C₁-C₁₂)-alkoxy, (C₁-C₁₂)-Alkoxy-amino, (C₁-C₁₂)-Alkoxy-N (C₁-C₆)-alkylamino, (C₁-C₁₂)-Alkoxy-N,N (C₁-C₆)-dialkylamino, Carbamoyloxy, N-(C₁-C₈)-Alkylcarbamoyloxy, N,N-Di-(C₁-C₈)-alkylcarbamoyl, N-(C₃-C₈)-Cycloalkylcarbamoyl, N-(C₆-C₁₂)-Arylamino, N-(C₇-C₁₁)-Aralkylamino, N-Alkyl-Aralkylamino, N-Alkyl-Arylamino, (C₃-C₈)-Cycloalkanoylamino, (C₁-6₈)-Alkanoylamino, (C₆-C₁₂)-Aroylamino, (C₇-C₁₁)-Aralkanoylamino, (C₁-C₈)-Alkanoyl-(C₁-C₈)-alkylamino, (C₃-C₈)-Cycloalkanoyl-(C₁-C₈)-alkylamino,(C₆-C₁₂)-Aroyl-(C₁-C₈)-alkylamino, (C₇-C₁₁)-Aralkanoyl-(C₁-C₈)-alkylamino,(C₁-C₈)-Alkylmercapto, (C₁-C₈)-Alkylsulfinyl, (C₁-C₈)-Alkylsulfonyl, (C₁-C₈)-Alkylcarbonyl,(C₃-C₈)-Cycloalkylcarbonyl, Nitro, Trifluormethyl, Phenylmercapto, Phenylsulfonyl, Phenylsulfinyl, Sulfamoyl, N-(C₁-C₆)-Alkylsulfamoyl, N,N-Di-(C₁-C₆)-alkylsulfamoyl, (C₁-C₈)-Alkyl-sulfonamido, Arylsulfonamido, wobei die in vorstehenden Substituenten vorhandenen Aryl- und Aralkylreste auch heterocyclischer Natur sein können und/oder, wie auch Alkyl, mit 1, 2, 3, 4 oder 5 gleichen oder verschiedenen Substituenten aus der Reihe Halogen, Cyano, Nitro, Trifluormethyl, (C₁-C₆)-Alkyl-Hydroxy, (C₁-C₆)-Hydroxyalkyl, (C₁-C₆)-Alkoxy, -O-[CH₂-]ₓC_{f}H_{(2f+1-g)}F_{g}, -OCF₂Cl, -O-CF₂-CHFCl, Trifluormethyl, (C₁-C₆)-Alkylmercapto, (C₁-C₆)-Alkylsulfinyl, (C₁-C₆)-Alkylsulfonyl, (C₁-C₆)-Alkylcarbonyl, (C₁-C₆)-Alkoxycarbonyl, Carbamoyl, N-(C₁-C₄)-Alkylcarbamoyl, N,N-Di-(C₁-C₄)-alkylcarbamoyl, (C₁-C₆)-Alkylcarbonyloxy, (C₃-C₈)-Cycloalkyl, Phenyl, Benzyl, Phenoxy, Benzyloxy, NR'-R'', Phenylmercapto, Phenylsulfonyl, Phenylsulfinyl, Sulfamoyl, N-(C₁-C₄)-Alkylsulfamoyl oder N,N-Di-(C₁-C₄)-alkylsulfamoyl, insbesondere bis zu mit 3 der vorstehend genannten gleichen oder verschiedenen Substituenten substituiert sind und eine CH₂-Gruppe der Alkylkette gegebenenfalls durch O, S, SO, SO₂ oder NR' ersetzt ist, oder mit
einem unsubstituierten oder substituierten (C₆-C₁₂)-Arylrest oder Heteroarylrest, der 1, 2, 3, 4 oder 5 gleiche oder verschiedene Substituenten aus der Reihe Halogen, Nitro, Cyano, Carboxyl, Hydroxy, Trifluormethyl, (C₁-C₆)-Hydroxyalkyl,-O-[CH₂]ₓC_{f}H_{(2f+1-g)}F_{g}, -OCF₂Cl, -OCF₂-CHFCl, (C₁-C₆)-Alkylmercapto, (C₁-C₆)-Alkylsulfinyl, (C₁-C₆)-Alkylsulfonyl, (C₁-C₈)-Alkoxy, (C₁-C₈)-Alkyl, (C₁-C₆)-Alkylcarbonyl, (C₁-C₆)-Alkoxycarbonyl, Carbamoyl, N-(C₁-C₄)-Alkylcarbamoyl, N,N-Di-(C₁-C₄)-alkylcarbamoyl, (C₁-C₆)-Alkylcarbonyloxy, (C₃-C₈)-Cycloalkyl, Phenyl, Benzyl, Phenoxy, Benzyloxy, NR'-R'', Phenylmercapto, Phenylsulfonyl, Phenylsulfinyl, Sulfamoyl, N-(C₁-C₄)-Alkylsulfamoyl, N,N-Di-(C₁-C₄)-alkylsulfamoyl, (C₁-C₈)-Alkoxycarbonyloxy, (C₁-C₈)-Alkoxy-(C₁-C₈)-alkoxycarbonyloxy, (C₆-C₁₂)-Aryloxycarbonyloxy, (C₇-C₁₁)-Aralkyloxycarbonyloxy, (C₇-C₁₁)-Aralkylcarbonyloxy, Cinnamoyl, Cinnamoyloxy, (C₆-C₁₂)-Arylcarbonyloxy, (C₃-C₈)-Alkenylcarbonyloxy, (C₃-C₈)-Alkinylcarbonyloxy, (C₃-C₈)-Cycloalkylcarbonyloxy, (C₁-C₁₂)-Alkoxy-(C₁-C₁₂)-alkoxy, (C₁-C₁₂)-Alkoxy-amino, (C₁-C₁₂)-Alkoxy-N-(C₁-C₆)-alkylamino, (C₁-C₁₂)-Alkoxy-N,N(C₁-C₆)-dialkylamino, Carbamoyloxy, N-(C₁-C₈)-Alkylcarbamoyloxy, N,N-Di-(C₁-C₈)-alkylcarbamoyl, N-(C₃-C₈)-Cycloalkylcarbamoyl, N-(C₆-C₁₂)-Arylamino, N-(C₇-C₁₁)-Aralkylamino, N-Alkyl-Aralkylamino, N-Alkyl-Arylamino, (C₃-C₈)-Cycloalkanoylamino, (C₁-C₈)-Alkanoylamino, (C₆-C₁₂)-Aroylamino, (C₇-C₁₁)-Aralkanoylamino, (C₁-C₈)-Alkanoyl-(C₁-C₈)-alkylamino, (C₃-C₈)-Cycloalkanoyl-(C₁-C₈)-alkylamino,(C₆-C₁₂)-Aroyl-(C₁-C₈)-alkylamino, (C₇-C₁₁)-Aralkanoyl-(C₁-C₈)-alkylamino,(C₁-C₈)-Alkylmercapto, (C₁-C₈)-Alkylsulfinyl, (C₁-C₈)-Alkylsulfonyl, (C₁-C₈)-Alkylcarbonyl,(C₃-C₈)-Cycloalkylcarbonyl, Nitro, Trifluormethyl, Phenylmercapto, Phenylsulfonyl, Phenylsulfinyl, Sulfamoyl, N-(C₁-C₆)-Alkylsulfamoyl, N,N-Di-(C₁-C₆)-alkylsulfamoyl, (C₁-C₈)-Alkyl-sulfonamido und Arylsulfonamido trägt, wobei die in vorstehenden Substituenten vorhandenen Aryl- und Aralkylreste auch heterocyclischer Natur sein können und/oder, wie auch Alkyl, mit 1, 2, 3, 4 oder 5 gleichen oder verschiedenen Substituenten aus der Reihe Halogen, Cyano, Nitro, Trifluormethyl, (C₁-C₆)-Alkyl, Hydroxy, (C₁-C₆)-Hydroxyalkyl, (C₁-C₆)-Alkoxy, substituiert sein können, oder mit
einem unsubstituierten oder substituierten (C₆-C₁₂)-Aryloxyrest, (C₇-C₁₁)-Aralkyloxyrest oder Heteroaryloxyrest, der 1, 2, 3, 4 oder 5 gleiche oder verschiedene Substituenten aus der Reihe Hydroxy, Halogen, Cyano, Nitro, Trifluormethyl, (C₁-C₆) Alkyl, (C₁-C₆)-Hydroxyalkyl, (C₁-C₆)Alkoxy, [CH₂-]ₓC_{f}H_{(2f+1-g)} F_{g}, -OCF₂-CHFCl, (C₁-C₆)-Alkylmercapto, (C₁-C₆)-Alkylsulfinyl, (C₁-C₆)-Alkylsulfonyl, (C₁-C₆)-Alkylcarbonyl, (C₁-C₆)-Alkoxycarbonyl, Carbamoyl, N-(C₁-C₄)-Alkylcarbamoyl, N,N-Di-(C₁-C₄)-alkylcarbamoyl, (C₁-C₆)-Alkylcarbonyloxy, (C₃-C₈)-Cycloalkyl, Carboxyl, Phenyl, Benzyl, Phenoxy, Benzyloxy, NR'-R'', Phenylmercapto, Phenylsulfonyl, Phenylsulfinyl, Sulfamoyl, N-(C₁-C₄)-Alkylsulfamoyl, N,N-Di-(C₁-C₄)-alkylsulfamoyl, Aminoalkyl, N-(C₁-C₈)-alkylamino-(C₁-C₁₂)alkyl oder N-Di-(C₁-C₈)-alkylamino-(C₁-C₁₂)-alkyl trägt, gegebenenfalls bis zu mit 3 der vorstehend genannten gleichen oder verschiedenen Substituenten substituiert ist und eine CH₂-Gruppe der Alkylkette gegebenenfalls durch O, S, SO, SO₂ oder NR' ersetzt ist, oder mit
einem Rest der allgemeinen Formel II

- O - R⁸ (II),

worin
- R⁸: eine über ihren Acylrest gebundene Aminosäure, ihr Derivat oder eine Alkoholschutzgruppe bedeutet,
- B: einen unsubstituierten oder substituierten (C₆-C₁₂)Arylrest, (C₇-C₁₁)Aralkylrest oder einen Heteroarylrest bedeutet, der einfach oder mehrfach, vorzugsweise ein- oder zweifach substituiert ist mit
Hydroxy, Halogen, Cyano, Carboxyl, Amino, (C₁-C₈)-Alkyl, (C₁-C₈)-Alkoxy, (C₁-C₈)-Alkoxycarbonyl, (C₁-C₈)-Alkylcarbonyl, (C₁-C₈)-Alkylcarbonyloxy, (C₁-C₈)-Alkylamino, Di-(C₁-C₈)-alkylamino, (C₁-C₆)-Hydroxyalkyl, -O-[CH₂-]ₓC_{f}H_{(2f+1-g)}F_{g}, -OCF₂Cl, -OCF₂-CHFCl, Carbamoyl, N-(C₁-C₈)-Alkylcarbamoyl, N,N-Di-(C₁-C₈)-alkylcarbamoyl, (C₁-C₈)-Alkylcarbonyloxy, (C₃-C₈)-Cycloalkyl, Phenyl, Benzyl, Phenoxy, Benzyloxy, Aminoalkyl, N-(C₁-C₈)-alkylamino (C₁-C₁₂)-alkyl oder N,N-Di-(C₁-C₈)-alkylamino-(C₁-C₁₂)-alkyl, (C₁-C₈)-Alkoxycarbonyloxy, (C₁-C₈)-Alkoxy-(C₁-C₈)-alkoxycarbonyloxy, (C₆-C₁₂)-Aryloxycarbonyloxy, (C₇-C₁₁)-Aralkyloxycarbonyloxy, (C₇-C₁₁)-Aralkylcarbonyloxy, Cinnamoyl, Cinnamoyloxy, (C₆-C₁₂)-Arylcarbonyloxy, (C₃-C₈)-Alkenylcarbonyloxy, (C₃-C₈)-Alkinylcarbonyloxy, (C₃-C₈)Cycloalkylcarbonyloxy, (C₁-C₁₂)-Alkoxy-(C₁-C₁₂)-alkoxy, (C₁-C₁₂)-Alkoxy-amino, (C₁-C₁₂)-Alkoxy-N (C₁-C₆)-alkylamino, (C₁-C₁₂)-Alkoxy-N,N (C₁-C₆)-dialkylamino, Carbamoyloxy, N-(C₁-C₈)-Alkylcarbamoyloxy, N,N-Di-(C₁-C₈)-alkylcarbamoyl, N-(C₃-C₈)-Cycloalkylcarbamoyl, N-(C₆-C₁₂)-Arylamino, N(C₇-C₁₁)-Aralkylamino, N-Alkyl-Aralkylamino, N-Alkyl-Arylamino, (C₃-C₈)-Cycloalkanoylamino, (C₁-C₈)-Alkanoylamino, (C₆-C₁₂)-Aroylamino, (C₇-C₁₁)-Aralkanoylamino, (C₁-C₈)-Alkanoyl-(C₁-C₈)-alkylamino, (C₃-C₈)-Cycloalkanoyl-(C₁-C₈)-alkylamino, (C₆-C₁₂)-Aroyl-(C₁-C₈)alkylamino, (C₇-C₁₁)-Aralkanoyl-(C₁-C₈)-alkylamino, (C₁-C₈)-Alkylmercapto, (C₁-C₈)-Alkylsulfinyl, (C₁-C₈)-Alkylsulfonyl, (C₁-C₈)-Alkylcarbonyl, (C₃-C₈)-Cycloalkylcarbonyl, Nitro, Trifluormethyl, Phenylmercapto, Phenylsulfonyl, Phenylsulfinyl, Sulfamoyl, N-(C₁-C₆)-Alkylsulfamoyl, N,N-Di-(C₁-C₆)-alkylsulfamoyl, (C₁-C₈)-Alkyl-sulfonamido, Arylsulfonamido,
- C: im Falle von X = -N(R⁷),einen unsubstituierten oder substituierten (C₁-C₁₂)Alkoxyrest, (C₃-C₈)-Cycloalkoryrest, (C₆-C₁₂)-Aryloxyrest oder einen (C₇-C₁₁)-Aralkyloxyrest bedeuten, der einfach oder mehrfach, vorzugsweise ein- und zweifach substituiert ist mit
Halogen, Trifluormethyl, (C₁-C₆)-Alkoxy, Hydroxy, (C₁-C₆)-Hydroxyalkyl, NR'R'' oder Cyano,
wobei jeweils
R' und R'' gleich oder verschieden sind und Wasserstoff, (C₆-C₁₂)-Aryl, (C₁-C₈)-Alkyl, (C₁-C₈)-Alkylcarbonyl, (C₇-C₁₁)Aralkylcarbonyl oder (C₆-C₁₂)-Arylcarbonyl bedeuten oder mit dem Stickstoff einen gesättigten heterocyclischen Ring bilden, vorzugsweise einen 5- oder 6-gliedrigen Ring,
und
R², R⁵ und R⁴ oder R³, sofern R⁴ oder R³ nicht schon oben definiert ist, gleich oder verschieden sind und
- D: Wasserstoff, wobei mindestens ein Rest R², R⁵ und R⁴ oder R³ ungleich Wasserstoff ist, Halogen, insbesondere Fluor, Chlor oder Brom, Cyano, Nitro, Trifluormethyl, (C₁-C₁₂)-Alkyl, -O-[CH₂-]ₓC₁H_{(2f+1-g)}F_{g}, -OCF₂Cl, -O-CF₂-CHFCl, (C₁-C₈)-Alkylmercapto, (C₁-C₈)-Alkylsulfinyl, (C₁-C₈)-Alkylsulfonyl, (C₁-C₈)-Alkylcarbonyl, Carbamoyl, N-(C₁-C₄)-Alkylcarbamoyl, N,N-Di-(C₁-C₄)-alkylcarbamoyl, (C₃-C₈)-Cycloalkyl, Phenylmercapto, Phenylsulfonyl, Phenylsulfinyl, (C₁-C₁₂)-Alkoxycarbanoyl, (C₁-C₁₂)-Alkylcarbanoyloxy, Amino, N-(C₁-C₁₀)-Alkylamino, Di-N,N-(C₁-C₁₀)-alkylamino, N,N-(C₃-C₈)-Alkandiylamino, wie z. B. Pyrollidino, Piperidino oder ihre heterocyclischen Derivate Morpholino, Thiomorpholino, N-(C₆-C₁₂)-Arylamino, N-(C₆-C₁₂)-Aryl-N-(C₁-C₁₀)-alkylamino, N-(C₇-C₁₁)-Aralkylamino, N-(C₇-C₁₁)-Aralkyl-N-(C₁-C₁₀)-alkylamino, (C₁-C₁₂)-Alkanoylamino, (C₃-C₈)-Cycloalkanoylamino(C₁-C₁₂)-Hydroxyalkanoylamino, (C₁C₈)-Alkoxy-(C₁-C₁₂)alkanoylamino, (C₆-C₁₂)-Arylcarbonylamino, (C₇-C₁₁)-Aralkylcarbonylamino, (C₁-C₈)-Alkoxycarbonyloxy, (C₁-C₈)-Alkoxy-(C₁-C₈)-alkoxycarbonyloxy, (C₆-C₁₂)-Aryloxycarbonyloxy, (C₇-C₁₁)-Aralkyloxy carbonyloxy, (C₇-C₁₁)-Aralkylcarbonyloxy, (C₆-C₁₂)-Arylcarbonyloxy, (C₃-C₈)-Alkenylcarbonyloxy, (C₃-C₈)-Alkinylcarbonyloxy, (C₃-C₈)-Cycloalkylcarbonyloxy, (C₁-C₁₂)-Alkoxy-(C₁-C₁₂)-alkoxy, (C₁-C₁₂)-Alkoxy-amino, (C₁-C₁₂)-Alkoxy-N-(C₁-C₆-alkylamino, (C₁-C₁₂)-Alkoxy-N,N(C₁-C₆)-dialkylamino, Carbamoyloxy, N-(C₁-C₈)-Alkylcarbamoyloxy, N,N-Di-(C₁-C₈)-alkylcarbamoyloxy, N-(C₃-C₈)-Cycloalkylcarbamoyloxy, NR'R'', (C₁-C₈)-Alkylmercapto, (C₁-C₈)-Alkylsulfinyl, (C₁-C₈)-Alkylsulfonyl, (C₁-C₈)-Alkylcarbonyl, (C₃-C₈)-Cycloalkylcarbonyl, Sulfamoyl, N-(C₁-C₆)-Alkylsulfamoyl, N,N-Di-(C₁-C₆)-alkylsulfamoyl, (C₁-C₈)-Alkylsulfonamido oder Arylsulfonamido bedeutet, wobei die in vorstehenden Substituenten vorhandenen Aryl- und Aralkylreste auch heterocyclischer Natur sein können und/oder, wie auch Alkyl, mit 1, 2, 3, 4 oder 5 gleichen oder verschiedenen Substituenten aus der Reihe Halogen, Cyano, Nitro, Trifluormethyl, (C₁-C₆)-Alkyl, Hydroxy, (C₁-C₆)-Hydroxyalkyl, (C₁-C₆)-Alkoxy, -O-[CH₂-]ₓC_{f}H_{(2f+1-g)}F_{g}, -OCF₂Cl, -O-CF₂-CHFCl, Trifluormethyl, (C₁-C₆)-Alkylmercapto, (C₁-C₆)-Alkylsulfinyl, (C₁-C₆)-Alkylsulfonyl, (C₁-C₆)-Alkylcarbonyl, (C₁-C₆)-Alkoxycarbonyl, Carbamoyl, N-(C₁-C₄)Alkylcarbamoyl, N,N-Di-(C₁-C₄)-alkylcarbamoyl, (C₁-C₆)-Alkylcarbonyloxy, (C₃-C₈)-Cycloalkyl, Phenyl, Benzyl, Phenoxy, Benzyloxy, NR'-R'', Phenylmercapto, Phenylsulfonyl, Phenylsulfinyl, Sulfamoyl, N-(C₁-C₄)-Alkylsulfamoyl oder N,N-Di-(C₁-C₄)-alkylsulfamoyl, insbesondere mit bis zu 3 der vorstehend genannten gleichen oder verschiedenen Substituenten substituiert sind und eine CH₂-Gruppe der Alkylkette gegebenenfalls durch O, S, SO, SO₂ oder NR' ersetzt ist,
oder
- E: einen Alkyl-, Alkenyl- oder Alkinylrest mit bis zu 9 C-Atomen bedeutet, welcher gegebenenfalls substituiert ist mit
1, 2, 3, 4 oder 5 gleichen oder verschiedenen Substituenten aus der Reihe Hydroxy, Halogen, Cyano, Nitro, Trifluormethyl, (C₁-C₆)-Hydroxyalkyl, (C₁-C₆)Alkoxy, [CH₂-]ₓC_{f}H_{(2f+1-g)}F_{g}, -OCF₂-CHFCl, (C₁-C₆)-Alkylmercapto, (C₁-C₆)-Alkylsulfinyl, (C₁₋ C₆)-Alkylsulfonyl, (C₁-C₆)-Alkylcarbonyl, (C₁-C₆)-Alkoxycarbonyl, Carbamoyl, N-(C₁-C₄)-Alkylcarbamoyl, N,N-Di-(C₁-C₄)-alkylcarbamoyl, (C₁-C₆)-Alkylcarbonyloxy, (C₃-C₈)-Cycloalkyl, Carboxyl, Phenyl, Benzyl, Phenoxy, Benzyloxy, Phenylmercapto, Phenylsulfonyl, Phenylsulfinyl, Sulfamoyl, N-(C₁-C₄)-Alkylsulfamoyl, N,N-Di-(C₁-C₄)-alkylsulfamoyl, Amino, N-(C₁-C₁₀)-Alkylamino, Di-N,N-(C₁-C₁₀)-alkylamino, N,N-(C₃-C₈)-Alkandiylamino, wie z. B. Pyrollidino, Piperidino oder ihre heterocyclischen Derivate Morpholino, Thiomorpholino, N-(C₆-C₁₂)-Arylamino, N-(C₆-C₁₂)-Aryl-N-(C₁-C₁₀)-alkylamino, N-(C₇-C₁₁)-Aralkylamino, N-(C₇-C₁₁)-Aralkyl-N-(C₁-C₁₁)-alkylamino, (C₁-C₁₂)-Alkanoylamino, (C₃-C₈)-Cyclo-alkanonylamino, (C₁-C₁₂)-Hydroxyalkanoylamino, (C₁-C₈)-Alkoxy-(C₁-C₁₂)-alkanoylamino, (C₆-C₁₂)-Arylcarbonylamino, (C₇-C₁₁)-Aralkylocarbonylamino, insbesondere mit bis zu 3 der vorstehend genannten gleichen oder verschiedenen Substituenten und eine CH₂-Gruppe der Alkylkette gegebenenfalls durch O, S, SO, SO₂ oder NR' ersetzt ist, oder mit
einem unsubstituierten oder substituierten (C₆-C₁₂)-Arylrest, (C₇-C₁₁)-Aralkylrest oder Heteroarylrest, der 1, 2, 3, 4 oder 5 gleiche oder verschiedene Substituenten aus der Reihe Hydroxy, Halogen, Cyano, Nitro, Trifluormethyl, (C₁-C₆)-Hydroxyalkyl, (C₁-C₆)-Alkyl, (C₁-C₆)Alkoxy, [CH₂-]ₓC_{f}H_{(2f+1-g)}F_{g,} -OCF₂-CHFCl, (C₁-C₆)-Alkylmercapto, (C₁-C₆)-Alkylsulfinyl, (C₁-C₆)-Alkylsulfonyl, (C₁-C₆)-Alkylcarbonyl, (C₁-C₆)-Alkoxycarbonyl, Carbamoyl, N-(C₁-C₄)-Alkylcarbamoyl, N,N-Di-(C₁-C₄)-alkylcarbamoyl, (C₁-C₆)-Alkylcarbonyloxy, (C₃-C₈)-Cycloalkyl, Carboxyl, Phenyl, Benzyl, Phenoxy, Benzyloxy, Phenylmercapto, Phenylsulfonyl, Phenylsulfinyl, Sulfamoyl, N-(C₁-C₄)-Alkylsulfamoyl, Amino, N-(C₁-C₁₀)-Alkylamino, Di-N,N-(C₁-C₁₀)-alkylamino, N,N-(C₃-C₈)-Alkandiylamino, wie z. B. Pyrollidino, Piperidino oder ihre heterocyclischen Derivate Morpholino, Thiomorpholino, N-(C₆-C₁₂)-Arylamino, N-(C₆-C₁₂)-Aryl-N-(C₁-C₁₀)-alkylamino, N-(C₇-C₁₁)aralkylamino, N-(C₇-C₁₁)-Aralkyl-N-(C₁-C₁₀)alkylamino, (C₁-C₁₂)-Alkanoylamino, (C₃-C₈)-Cyclo-alkanonylamino, (C₁-C₁₂)-Hydroxyalkanoylamino, (C₁-C₈)-Alkoxy-(C₁-C₁₂)-alkanoylamino, (C₆-C₁₂)-Arylcarbonylamino, (C₇-C₁₁)-Aralkylcarbonylamino, insbesondere mit bis zu 3 der vorstehend genannten gleichen oder verschiedenen Substituenten und eine CH₂-Gruppe der Alkylkette gegebenenfalls durch O, S, SO, SO₂ oder NR' ersetzt ist, oder
- F: einen substituierten oder unsubstituierten (C₆-C₁₂)-Arylrest, (C₇-C₁₁)-Aralkylrest oder Heteroarylrest, wobei der genannte Aryl- und Heteroarylrest, insbesondere Phenyl, Naphthyl, Thienyl, Furyl, Pyrrolyl oder Pyridin bedeutet, und der weiterhin
1,2,3,4 oder 5 gleiche oder verschiedene Substituenten aus der Reihe Hydroxy, Halogen, Cyano, Nitro, (C₁-C₆)-Alkyl, (C₁-C₆)-Alkoxy, Carboxyl, Trifluormethyl, (C₁-C₆)-Hydroxyalkyl, -O-[CH₂-]ₓC_{f}H_{(2f+1g})F_{g}, -OCF₂Cl, -OCF₂-CHFCl, (C₁-C₆)-Alkylmercapto, (C₁-C₆)-Alkylsulfonyl, (C₁-C₆)-Alkylsulfinyl, (C₁-C₆)Alkylcarbonyl, (C₁-C₆)-Alkoxycarbonyl, Carbamoyl, N-(C₁-C₄)-Alkylcarbamoyl, N,N-Di-(C₁-C₄)-alkylcarbamoyl, (C₁-C₆)-Alkylcarbonyloxy, (C₃-C₈)-Cycloalkyl, Phenyl, Benzyl, Phenoxy, Benzyloxy, Amino, N-(C₁-C₁₀)-Alkylamino, Di-N,N-(C₁-C₁₀)-alkylamino, N,N-(C₃-C₈)-Alkandiylamino, wie z. B. Pyrollidino, Piperidino, Morpholino, Thiomorpholino, (C₁-C₁₀)-Alkanoylamino, (C₆-C₁₂)-Arylcarbonylamino, (C₇-C₁₁)-Aralkylcarbonylamino, Phenylmercapto, Phenylsulfonyl, Phenylsulfinyl, Sulfamoyl, N-(C₁-C₄)-Alkylsulfamoyl oder N,N-Di-(C₁-C₄)-alkylsulfamoyl, insbesondere bis zu 3 der vorstehend genannten gleichen oder verschiedenen Substituenten im Arylteil trägt, wobei eine CH₂-Gruppe der Arylkette gegebenenfalls durch O, S, SO, SO₂ oder NR' ersetzt ist, oder
- G: einen Substituenten der Formeln -OR⁹ oder -N(R⁹)₂ ist, wobei
- R⁹: Wasserstoff, Alkyl, Alkenyl oder Alkinyl jeweils mit bis zu 9 C-Atomen, einen (C₆-C₁₂)-Arylrest oder einen Heteroarylrest bedeutet, der
1,2,3,4 oder 5 gleiche oder verschiedene Substituenten aus der Reihe Hydroxy, Halogen, Hydroxy, Cyano, Nitro, Carboxyl, (C₁-C₆)-Alkyl, (C₁-C₆)-Alkoxy, Trifluormethyl, (C₁-C₆)-Hydroxyalkyl, -O-[CH₂-]ₓC_{f}H_{(2f+1g)}F_{g}, -OCF₂Cl, -OCF₂-CHFCl, (C₁-C₆)-Alkylmercapto, (C₁-C₆)Alkylsulfonyl, (C₁-C₆)-Alkylsulfinyl, (C₁-C₆)-Alkylcarbonyl, (C₁-C₆)-Alkoxycarbonyl, Carbamoyl, N-(C₁-C₄)-Alkylcarbamoyl, N,N-Di-(C₁-C₄)-alkylcarbamoyl, (C₁-C₆)-Alkylcarbonyloxy, (C₃-C₈)-Cycloalkyl, Phenyl, Benzyl, Phenoxy, Bezyloxy, Amino, N-(C₁-C₁₀)-Alkylamino, Di-N,N-(C₁-C₁₀)-alkylamino, N,N-(C₃-C₈)-Alkandiylamino, wie z. B. Pyrollidino, Piperidino, Morpholino, Thiomorpholino, (C₁-C₁₀)-Alkanoylamino, (C₆-C₁₂)-Arylcarbonylamino, (C₇-C₁₁)-Aralkylcarbonylamino, Phenylmercapto, Phenylsulfonyl, Phenylsulfinyl, Sulfamoyl, N-(C₁-C₄)-Alkylsulfamoyl oder N,N-Di-(C₁-C₄)-alkylsulfamoyl, insbesondere bis zu 3 der vorstehend genannten gleichen oder verschiedenen Substituenten im Arylteil trägt, wobei eine CH₂-Gruppe der Arylkette gegebenenfalls durch O, S, SO, SO₂ oder NR' ersetzt ist,
und
- n =: 0 der 1,
- f =: 1 bis 8, vorzugsweise 1 bis 5,
- g =: 0,1 bis (2f + 1),
- x: 0, 1, 2 oder 3, vorzugsweise 0 oder 1 ist,
zuzüglich alle Derivate, die in ihren Amino- oder Hydroxygruppen eine entsprechende Schutzgruppe aufweisen, sowie die physiologisch wirksamen Salze.

Unter Aryl-, Aryloxy-, Heteroaryl- bzw. Heteroaryloxyerbindungen versteht man insbesondere Phenyl- und Naphtyl- bzw. unsubstituierte 5- und 6-gliedrige heteroaromatische Ringe mit 1, 2 oder 3 Stickstoff- und/oder Sauerstoff und/oder Schwefelatomen, wie Pyridyl-, Pyridazyl-, Pyrimidyl-, Pyrazyl-, Imidazolyl-, Triazolyl-, Thienyl-, Oxazolyl-, und Thiazolyl-Derivate, und deren benzoannellierte Derivate. Unter dem Rest (C₇-C₁₁)-Aralkyloxy ist vorzugsweise ein substituierter Phenylalkyloxyrest der Formel III 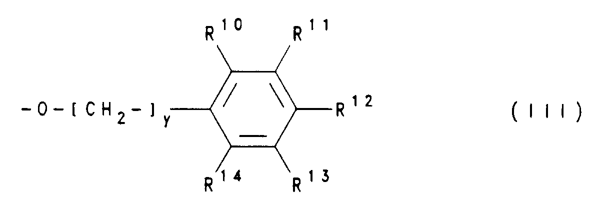
zu verstehen,
worin R¹⁰, R¹¹, R¹², R¹³ und R¹⁴ gleich oder verschieden sind und Wasserstoff, Halogen, Cyano, Nitro, Trifluormethyl, (C₁-C₆)-Alkyl, (C₁-C₆)-Alkoxy, -O-[CH₂-]ₓC_{f}H_{(2f+1-g)}F_{g}, -OCF₂Cl, -O-CF₂-CHFCl, (C₁-C₆)-Alkylmercapto, (C₁-C₆)-Alkylsulfinyl, (C₁-C₆)-Alkylsulfonyl, (C₁-C₆)-Alkylcarbonyl, (C₁-C₆)-Alkoxycarbonyl, Carbamoyl, N-(C₁-C₄)-Alkylcarbamoyl, N,N-Di-(C₁-C₄)-alkylcarbamoyl, (C₁-C₆)-Alkylcarbonyloxy, (C₃-C₈)-Cycloalkyl, Phenyl, Benzyl, Phenoxy, Benzyloxy, NR'R'', wie Amino, Anilino, N-Methylanilino, Phenylmercapto, Phenylsulfonyl, Phenylsulfinyl, Sulfamoyl, N-(C₁-C₄)-Alkylsulfamoyl oder N,N-Di-(C₁-C₄)-alkylsulfamoyl bedeuten, oder zwei benachbarte Substituenten gemeinsam eine Kette -[CH₂-]ₙ oder -CH=CH-CH=CH-bedeuten, wobei eine CH₂-Gruppe der Kette gegebenenfalls durch O, S, SO, SO₂ oder NR' ersetzt ist, Y = 1, 2, 3 oder 4, vorzugsweise 0 und 1 bedeutet und die übrigen der Substituenten R¹⁰, R¹¹, R¹², R¹³ und R¹⁴ wie vorstehend definiert sind.

Von den genannten Aminosäuren werden insbesondere die natürlichen α-Aminosäuren bevorzugt.

Unter Aminoschutzgruppen versteht man insbesondere solche Gruppen, die in R. Geiger und W. König "The Peptides" Volume 3, "Protection of Functional Groups in Peptide Synthesis", E. G. Gross, J. Meienhofer Edit, Academic Press, New York (1981), insbesondere Seiten 7 - 46, beschrieben sind.

Ebenso werden solche Gruppen in A. Hubbuch, Schutzgruppen in der Peptidsynthese, Kontakte 3/79, Seiten 14-23 beschrieben.

Insbesondere bevorzugt sind folgende Aminoschutzgruppen:
Acetamidomethyl,
1-Adamantyloxycarbonyl,
1-(1-Adamantyl)-1-methyl-ethoxycarbonyl,
Allyloxycarbonyl,
tert.-Buryloxycarbonyl,
1-(4-Biphenylyl)-1-methyl-ethoxycarbonyl,
Dicyclohexylcarbodiimid,
α,α-Dimethyl-3,5-dimethoxybenzyloxycarbonyl,
4-Dihydroxyborylbenzyloxycarbonyl,
9-Fluorenylmethyloxycarbonyl,
1 Hydroxybenzotriazol,
3-Hydroxy-4-oxo-3,4-dihydro-1,2,3-benzotriazin,
Isobornyloxycarbonyl,
1-Methyl-cyclobutyloxycarbonyl,
4-Methoxybenzyloxycarbonyl,
Methylsulfonylethyloxycarbonyl,
4-Pyridylmethyloxycarbonyl,
2,2,2-Trichloro-tert.-butyloxycarbonyl,
Benzyloxycarbonyl,
halogensubstituiertes Benzyloxycarbonyl,
4-Nitro-benzyloxycarbonyl,
2-Phosphonoethyloxycarbonyl,
Phenylsulfonylethoxycarbonyl,
Toluolsulfonylethoxycarbonyl,
2,3,5-Trimethyl-4-methoxy-phenylsulfonyl,
Benzotriazol-1-yl-oxy-tris(dimethylamino)phosphonium-hexafluorophosphat.

Von den Verbindungen der allgemeinen Formel I, deren Aminogruppen geschützt sind, werden bevorzugt diejenigen, deren geschützte Aminogruppe Teil dieser Aminosäure R⁸ sind.

Als Alkohol-Schutzgruppe kommen insbesondere substituierte oder unsubstituierte Methylether, Ethylether, Benzylether, Silylether, Ester, Carbonate oder Sulfonate in Frage.

Hierunter fallen folgende Verbindungen:
Als substituierte Methylether:
Methoxymethyl, Methylthiomethyl, t-Butylthiomethyl, (Phenyldimethylsilyl)methoxymethyl, Benzyloxymethyl, p-Methoxybenzyloxymethyl, (4-Methoxyphenoxy)-methyl, Guaiacolmethyl, t-Butoxymethyl, 4-Pentenyloxymethyl, Siloxymethyl, 2-Methoxyethoxymethyl, 2,2,2-Trichlorethorymethyl, Bis(2-chlorethoxy)methyl, 2-(Trimethylsilyl)ethoxymethyl, Tetrahydropyranyl, 3-Bromotetrahydropyranyl, Tetrahydrothiopyranyl, 1-Methoxycyclohexyl, 4-Methoxytetrahydropyranyl, 4-Methoxytetrahydrothiopyranyl, 4-Methoxytetrahydrothiopyranyl-S,S-Dioxo, 1-[2-Chlor-4-methyl)-phenyl]-4-methoxypiperidin-4-yl, 1,4-Dioxan-2yl, Tetrahydrofuranyl, Tetrahydrothiofuranyl.

Als substituierte Ethylether:
1-Ethoxyethyl, 1-(2-Chlorethoxy)ethyl, 1-Methyl-1-methoxyethyl, 1-Methyl-1-benzyloxyethyl, 1-Methyl-1-benzyloxy-2-fluoroethyl, 2,2,2-Trichlorethyl, 2-Trimethylsilylethyl, 2-(Phenylselenyl)ethyl, t-Butyl, Allyl, p-Chlorphenyl, p-Methoxyphenyl, 2,4-Dinitrophenyl, Benzyl.

Als substituierte Benzylether:
p-Methoxybenzyl, 3,4-Dimethoxybenzyl, o-Nitrobenzyl, p-Nitrobenzyl, p-Halogenbenzyl, 2,6-Dichlorbenzyl, p-Cyanobenzyl p-Phenylbenzyl, 2- und 4-Picolyl, 3-Methyl-2-picolyl-N-oxido, Diphenylmethyl, p,p'-Dinitrobenzhydryl, Triphenylmethyl, α-Naphthyldiphenylmethyl, p-Methoxyphenyldiphenylmethyl, Di(p-methoxyphenyl)-phenylmethyl, Tri(p-methoxyphenyl)methyl, 4-(4'-Bromphenacyloxy)phenyldiphenylmethyl, 4,4',4''-Tris(4,5-dichlorphthalimidophenyl)methyl, 4,4',4''-Tris(levulinooxyphenyl)methyl, 4,4',4''-Tris(benzoyloxyphenyl)methyl, 3-(Imidazol-1,4'-methyl)bis(4',4''-dimethoxyphenyl)-methyl, 1,1-Bis(4-methoxyphenyl)-1'-pyrenylmethyl, 9-Anthryl, 9-(9-Phenyl)xanthenyl, 9-(9-Phenyl-10-oxo)anthryl.

Als Silylether:
Trimethylsilyl, Triethylsilyl, Triisopropylsilyl, Dimethylisopropylsilyl, Diethylisopropylsilyl, Dimethylthexylsilyl, t-Butyldimethylsilyl, t-Butyldiphenylsilyl, Tribenzylsilyl, Tri-p-xylylsilyl, Triphenylsilyl, Diphenylmethylsilyl, t-Butylmethoxyphenylsilyl.

Als Ester:
Formate, Benzoylformate, Acetate, Chloroacetat, Dichloroacetat, Trichloroacetat, Trifluoroacetat, Methoxyacetat, Triphenylmethoxyacetat, Phenoxyacetat, p-Chlorphenoxyacetat, p-P-Phenylacetat, 3-Phenylpropionat, 4-Oxopentanoat (Levulinat), 4,4-(Ethylendithio)pentanoat, Pivaloat, Adamantoat, Crotonat, 4-Methoxycrotonat, Benzoat, p-Phenylbenzoat, 2,4,6-Trimethylbenzoat (Mesitoat).

Als Carbonate:
Methyl, 9-Fluorenylmethyl, Ethyl, 2,2,2,-Trichlorethyl, 2-(Trimethylsilyl)ethyl, 2-(Phenylsulfonyl)ethyl, 2-(Triphenylphosphonio)ethyl, Isobutyl, Vinyl, Allyl, p-Nitrophenyl, Benzyl, p-Methoxybenzyl, 3,4-Dimethoxybenzyl, o-Nitrobenzyl, p-Nitrobenzyl, S-Benzyl Thioxarbonate, 4-Ethoxy-1-naphthyl, Methyl Dithiocarbonate.

Weitere Ester:
2,6-Dichlor-4-methylphenoxyacetat, 2,6-Dichlor-4-(1,1,3,3-tetramethylbutyl)phenoxyacetat, 2,4-Bis(1,1-dimethylpropyl)phenoxyacetat, Chlordiphenylacetat, Isobutyrat, Monosuccinat, (E)-2-Methyl-2-butenoat (Tigloat), O-(Methoxycarbonyl)benzoat, p-P-Benzoat, α-Naphthoat, Nitrat, Alkyl N,N,N', N'-Tetramethylphosphorodiamidat, N-Phenylcarbamat, Borate, Dimethylphosphinothioyl, 2,4-Dinitrophenylsulfenat.

Als Sulfonate:
Sulfate, Methansulfonat (Mesylat), Benzylsulfonat,
Tosylate.

Insbesondere bevorzugt sind folgende Schutzgruppen:
(C₁-C₆)-Alkanoyl, (C₁-C₈)-Alkylcarbamoyl, Di-(C₁-C₈)-alkylcarbamoyl, N-(C₃-C₈)-cycloalkylcarbamoyl, (C₁-C₆)-Alkoxycarbonyl, (C₆-C₁₂)-Aryloxycarbonyl, (C₇-C₁₁)Aralkyloxycarbonyl, insbesondere Benzyloxycarbonyl, (C₆-C₁₂)-Arylcarbonyl, (C₇-C₁₁)-Aralkylcarbonyl, (C₁-C₆)-Alkyl, (C₁-C₆)-Alkoxy-(C₁-C₆)-alkyl, Carbamoyl-(C₁-C₆)-alkylester, (C₁-C₁₀)-Acyloxy-(C₁-C₆)-alkyl, vorzugsweise (C₁-C₁₀)-Alkanoyloxy-(C₁-C₆)alkyl, Benzyloxy-(C₁-C₆)-alkyl, Benzyloxycarbonyloxy-(C₁-C₆)-alkyl, (C₁-C₆)-Alkoxycarbonyloxy-(C₁-C₆)-alkyl, Aminosäureester oder Tetrahydropyranyl.

Bevorzugt sind Verbindungen der allgemeinen Formel I, worin
- R¹ und R³ oder R⁴: -C(O)-X-R⁶ bedeutet, wobei
- X: -N(R⁷)- ist.

Weiterhin sind bevorzugt Verbindungen der allgemeinen Formel I, bei denen
- R⁶: Wasserstoff oder Methyl ist und
- R⁷: die oben angegebene Bedeutung hat oder
- R⁶ und R⁷: Wasserstoff und/oder Methyl ist, sofern zumindest eine Gruppe R¹, R³ oder R⁴ einen Rest -C(O)-N(R⁷)-R⁶ bedeutet, worin R⁶ und/oder R⁷ die oben angegebene Bedeutung hat.

Sofern R⁶ bzw. R⁷ nicht für Wasserstoff oder Methyl gemäß der oben bevorzugten Ausführungsform steht, wird folgendes bevorzugt, worin
- A: einen verzweigten oder unverzweigten (C₁-C₁₂)-Alkylrest bedeutet, der unsubstituiert ist oder einfach oder mehrfach substituiert ist mit
Halogen, insbesondere Fluor, Chlor, Brom, Hydroxy, Cyano, Carboxyl, (C₁-C₄)-Alkoxy, (C₁-C₄)-Alkoxycarboxyl, (C₁-C₈)-Alkoxycarbonyloxy, (C₁-C₈)-Alkoxy-(C₁-C₈)-alkoxycarbonyloxy, (C₆-C₁₂)-Aryloxycarbonyloxy, (C₇-C₁₁)-Aralkyloxycarbonyloxy, (C₇-C₁₁)-Aralkylcarbonyloxy, (C₇-C₁₁)-Arylcarbonyloxy, (C₃-C₈)-Cycloalkylcarbonyloxy, (C₁-C₁₂)-Alkoxy-(C₁-C₁₂)-alkoxy, Carbamoyloxy, N-(C₁-C₈)-Alkylcarbamoyloxy, N,N-Di (C₁-C₈)-alkylcarbamoyl, N-(C₃-C₈)-Cycloalkylcarbamoyl, N-(C₇-C₁₁)-Aralkylcarbamoyloxy, N (C₆-C₁₂)-Arylcarbamoyloxy, wobei die in vorstehenden Substituenten vorhandenen Aryl- und Aralkylreste auch heterocyclischer Natur sein können und/oder wie auch Alkyl mit 1 oder 2 gleichen oder verschiedenen Substituenten aus der Reihe Halogen, Trifluormethyl, Hydroxy, (C₁-C₃)-Alkyl, (C₁-C₃)-Hydroxyalkyl, (C₁-C₉)-Alkoxy, -O-[CH₂-]ₓC_{f}H_{(2f+1-g)}F_{g}, -OCF₂Cl, -O-CF₂-CHFCl, - (C₁-C₃)-Alkoxycarbonyl, Carbamoyl, (C₁-C₆)-Alkylcarbonyloxy, (C₃-C₈)-Cycloalkyl, Phenyl, Benzyl, Phenoxy, Benzyloxy, substituiert sind, oder mit
einem unsubstituierten oder substituierten (C₆-C₁₂)-Arylrest oder Heteroarylrest, der 1 oder 2 gleiche oder verschiedene Substituenten aus der Reihe Halogen, Nitro, Cyano, Carboxyl, Hydroxy, Trifluormethyl, (C₁-C₃)-Hydroxyalkyl, (C₁-C₃)-Alkoxycarbonyl, Carbamoyl, NR'R'', N-(C₁-C₄)-Alkylcarbamoyl, N,N-Di-(C₁-C₄)-alkylcarbamoyl, (C ₁-C₈)-Alkoxy-(C₁-C₈)-Alkyl, (C₁-C₃)-Alkylcarbonyloxy, Aminoalkyl oder N-(C₁-C₄)-alkylamino-(C₁-C₆)-alkyl trägt, wobei R' und R'' gleich oder verschieden sind und Wasserstoff, (C₆-C₁₂)-Aryl, oder (C₁-C₄)-Alkyl bedeuten, oder mit
einem unsubstituierten oder substituierten (C₆-C₁₀)-Aryloxyrest oder (C₇-C₁₁)-Aralkyloxyrest, der 1 oder 2 gleiche oder verschiedene Substituenten aus der Reihe Hydroxy, Halogen, Trifluormethyl, (C₁-C₃)-Alkyl, (C₁-C₃)-Hydroxyalkyl, (C₁-C₃)-Alkoxy, (C₁-C₃)-Alkylmercapto, (C₁-C₃)-Alkylsulfinyl, (C₁-C₃)-Alkylsulfonyl, (C₁-C₃)-Alkylcarbonyl, (C₁-C₃)-Alkoxycarbonyl, Carbamoyl, N-(C₁-C₄)-Alkylcarbamoyl, N,N-Di-(C₁-C₄)-alkylcarbamoyl, (C₁-C₃)-Alkylcarbonyloxy, NR'R'' trägt, wobei R' und R'' gleich oder verschieden sind und Wasserstoff, (C₆-C₁₀)-Aryl oder (C₁-C₄)-Alkyl bedeuten, oder mit
einem Rest der allgemeinen Formel II

- O - R⁸ (II)

worin
- R⁸: eine über ihren Acylrest gebundene Aminosäure oder ein Derivat hiervon bedeutet
- B: einen (C₆-C₁₂)Aryl- oder (C₇-C₁₁)Aralkylrest, vorzugsweise Phenyl, Benzyl und Phenethyl, bedeuten, welche unsubstituiert oder einfach substituiert sind mit Halogen, Cyano, Carboxyl, Hydroxy, (C₁-C₈)-Alkyl, (C₁-C₈)-Alkoxy, (C₁-C₈)-Alkylcarbonyl, (C₁-C₄)-Alkylcarbonyloxy, (C₁-C₄)-Alkoxycarbonyl, (C₁-C₄)-Hydroxyalkyl, Amino,
(C₁-C₅)-Alkylamino, Di-(C₁-C₅)alkylamino, (C₁-C₅)-Alkanoylamino, Carbamoyl, N-(C₁-C₄)-Alkylcarbamoyl, N,N-Di-(C₁-C₄)-alkylcarbamoyl, Carbamoyl, N-(C₁-C₄)-Alkylcarbamoyloxy, N,N-Di-(C₁-C₄)-alkylcarbamoyloxy, oder
- C: einen unsubstituierten (C₁-C₆)-Alkoxyrest, (C₃-C₈)Cycloalkoxyrest, (C₆-C₁₂)-Aryloxyrest oder
(C₇-C₁₁)-Aralkyloxyrest bedeuten.

Insbesondere bevorzugt sind Verbindungen der allgemeinen Formel I, in denen R⁶ und R⁷, sofern diese nicht für Wasserstoff oder Methyl, wie oben dargestellt, stehen,
- A: einen unverzweigten (C₁-C₁₂)-Alkylrest bedeutet, der unsubstituiert oder einfach substituiert ist mit
Hydroxy, Halogen, (C₁-C₈)-Alkoxy, (C₁-C₈)-Alkoxycarbonyl, (C₁-C₈)-Alkoxycarbonyloxy, (C₁-C₆)-Alkoxy-(C₁-C₈)-alkoxycarbonyloxy, (C₆-C₁₂)-Aryloxycarbonyloxy, (C₇-C₁₁)-Aralkyloxycarbonyloxy, (C₇-C₁₁)-Aralkylcarbonyloxy, (C₆-C₁₂)-Arylcarbonyloxy, (C₃-C₈)-Cycloalkylcarbonyloxy, (C₁-C₁₂)-Alkoxy-(C₁-C₁₂)-alkoxy, (C₁-C₁₂)-Alkoxy-amino, (C₁-C₁₂)-Alkoxy-N-(C₁-C₆)-alkylamino, (C₁-C₁₂)-Alkoxy-N,N-(C₁-C₆)-dialkylamino, N,N-Di-(C₁-C₈)-alkylcarbamoyl, N-(C₃-C₈)-Cycloalkylcarbamoyl, N-(C₇-C₁₁)-Aralkylcarbonyloxy, N-(C₆-C₁₂)-Arylcarbanoyloxy, (C₁-C₅)-Alkanoylamino, (C₃-C₆)-Cycloalkanoylamino, (C₆-C₁₂)-Aroylamino, (C₇-C₁₁)-Aralkamoylamino, wobei Alkyl, Aryl, Aryloxy, Aralkyl oder Aralkyloxy, ihrerseits substituiert sind mit Hydroxy, Halogen, insbesondere Fluor, (C₁-C₃)Alkyl, (C₁-C₃)Alkoxy, oder mit
einem unsubstituierten oder einfach mit einer Hydroxy-Gruppe substituierten Phenylrest, einem unsubstituierten oder substituierten Phenoxy- oder Benzyloxyrest, substituiert mit Hydroxy, Halogen, (C₁-C₄) Alkoxy, oder mit
einem Rest der allgemeinen Formel II

- O - R⁸ (II)

worin R⁸ eine über ihren Acylrest gebundene Aminosäure oder ihr an der Aminogruppe substituiertes Derivat bedeutet oder
- B: einen (C₆-C₁₂)-Aryl- oder (C₇-C₁₁)-Aralkylrest, vorzugsweise Phenyl, Benzyl und Phenethyl, bedeuten, welcher unsubstituiert oder einfach substituiert ist mit Hydroxy.

Bezüglich der Substituenten R², R⁵ und R⁴ oder R³, sofern R³ bzw. R⁴ nicht schon wie oben angegeben definiert ist, sind Verbindungen bevorzugt, in denen bevorzugt maximal zwei der drei Reste ungleich Wasserstoff sind, besonders bevorzugt maximal einer der Reste ungleich Wasserstoff ist.

Weiterhin bevorzugt sind Verbindungen, in denen unabhängig voneinander R², R⁵, und R⁴ oder R³ dann ungleich Wasserstoff sind, wenn diese maximal einem weiteren Substituenten R¹ und R³ oder R⁴ direkt benachbart sind.

R², R⁵ und R⁴ oder R³, sofern R⁴ oder R³ nicht schon oben definiert sind, und nicht Wasserstoff sein sollen, stehen bevorzugt für
- D: Halogen, insbesondere Fluor, Chlor oder Brom, Cyano, Nitro, Trifluormethyl, (C₁-C₁₂)-Alkyl, -O-[CH₂-]ₓC_{f}H_{(2f+g)}F_{g}, -OCF₂Cl, -O-CF₂-CHFCl, (C₁-C₈)Alkylcarbonyl, Carbamoyl, N-(C₁-C₄)Alkylcarbamoyl, N,N-Di-(C₁-C₄)alkylcarbamoyl, (C₃-C₈)-Cycloalkyl, (C₁-C₁₂)-Alkorycarbonyl, (C₁-C₁₂)-Alkylcarbonyloxy, Amino, N-(C₁-C₁₀)-Alkylamino, Di-N,N-(C₁-C₁₀)-alkylamino, N,N-(C₃-C₈)-Alkandiylamino, wie z. B. Pyrollidino, Piperidino oder ihre heterocyclischen Derivate Morpholino, Thiomorpholino, N-(C₆-C₁₂)-Arylamino, N-(C₆-C₁₂)-Aryl-N-(C₁-C₈)-alkylamino, N-(C₇-C₁₁)-Aralkylamino, N-(C₇-C₁₁)-Aralkyl-N-(C₁-C₁₀)-alkylamino, (C₆-C₁₂)-Arylcarbonylamino, (C₇-C₁₁)Aralkylcarbonylamino, (C₁-C₆)-Alkoxycarbonyloxy, (C₆-C₁₂)-Aryloxycarbonyloxy, (C₇-C₁₁)-Aralkylcarbonyloxy, (C₆-C₁₂)-Arylcarbonyloxy, (C₃-C₈)-Alkenylcarbonyloxy, (C₃-C₈)-Cycloalkylcarbonyloxy, (C₁-C₁₂)-Alkoxy-(C₁-C₁₂)-alkoxy, (C₁-C₁₂)-Alkoxy-amino, (C₁-C₁₂-Alkoxy-N-(C₁-C₆)-alkylamino, (C₁-C₁₂)-Alkoxy-N,N-(C₁-C₆)-dialkylamino, Carbamoyloxy, N-(C₁-C₆)Alkylcarbamoyloxy, N,N-Di-(C₁-C₈)-alkylcarbamoyloxy, N-(C₃-C₈)-Cycloalkylcarbamoyloxy, NR'R'', (C₁-C₈)-Alkylmercapto, (C₁-C₆)-Alkylsulfinyl, (C₁-C₈)-Alkylsufonyl, (C₁-C₈)-Alkylcarbonyl, (C₃-C₈)-Cycloalkylcarbonyl, wobei die in vorstehenden Substituenten vorhandenen Aryl-und Aralkylreste auch heterocyclischer Natur sein können und/oder, wie auch Alkyl, mit 1,2 oder 3 gleichen oder verschiedenen Substituenten aus der Reihe Halogen, Trifluormethyl, (C₁-C₆)-Alkyl Hydroxy, (C₁-C₆)-Hydroxyalkyl, (C₁-C₆)-Alkoxy, -O-[CH₂-]ₓC_{f}H_{(2f+1-g)}F_{g}, (C₃-C₈)-Cycloalkyl, Phenyl, Benzyl, Phenoxy, Benzyloxy, NR'-R'', Phenylmercapto, Phenylsulfonyl, Phenylsulfinyl, Sulfamoyl, N-(C₁-C₄)-Alkylsulfamoyl oder N,N-Di-(C₁-C₄)-alkylsulfamoyl,
oder
- E: einen Alkyl- oder Alkenylrest mit bis zu 5 C-Atomen bedeutet, welcher gegebenenfalls substituiert ist mit 1, 2 oder 3 gleichen oder verschiedenen Substituenten aus der Reihe Hydroxy, Halogen, Cyano, Nitro, Trifluormethyl (C₁-C₆)-Hydroxyalkyl, (C₁-C₆)Alkoxy, [CH₂]ₓC_{f}H_{(2f+1-g)}F_{g}, -OCF₂-CHFCl, (C₁-C₆)-Alkylcarbonyl, (C₁-C₆)-Alkoxycarbonyl, Carbamoyl, N-(C₁-C₄)-Alkylcarbamoyl, N,N-Di-(C₁-C₄)-alkylcarbamoyl, (C₁-C₆)-Alkylcarbonyloxy, (C₃-C₈)-Cycloalkyl, Carboxyl, Phenyl, Benzyl, Phenoxy, Benzyloxy, Amino, N-(C₁-C₆)-Alkylamino, Di-N,N-(C₁-C₆)-alkylamino, N,N-(C₃-C₈)-Alkandiylamino, wie z. B. Pyrollidino, Piperidino, N-(C₆-C₁₂)-Arylamino, N-(C₆-C₁₂)-Aryl-N-(C₁-C₆)-alkylamino, N-(C₇-C₁₁)-Aralkylamino, N-(C₇-C₁₁)-Aralkyl-N-(C₁-C₁₀)-alkylamino, (C₁-C₆)-Alkanoylamino, (C₁-C₈)-Alkoxy-(C₁-C₆)-alkanoylamino, (C₆-C₁₂)-Arylcarbonylamino, (C₇-C₁₁)-Aralkylocarbonylamino, oder mit
einem unsubstituierten oder substituierten (C₆-C₁₂)-Arylrest, (C₇-C₁₁)-Aralkylrest oder Heterarylrest, der 1,2 oder 3 gleiche oder verschiedene Sustituenten aus der Reihe Hydroxy, Halogen, Cyano, Nitro, Trifluormethyl, (C₁-C₆)-Hydroxyalkyl, (C₁-C₆)-Alkoxy,(C₁-C₆)-Alkoxy, [CH₂-]ₓC_{f}H_{(2f+1-g)}F_{g},(C₁-C₆)-Alkylcarbonyl, (C₁-C₆)-Alkoxycarbonyl, Carbamoyl, N(C₁-C₄)Alkylcarbamoyl, N,N-Di-(C₁-C₄)-alkylcarbamoyl, (C₁-C₆)-Alkylcarbonyloxy, (C₃-C₈)-Cycloalkyl, Carboxyl, Phenyl, Benzyl, Phenoxy, Benzyloxy, Amino, N-(C₁-C₆)-Alkylamino, Di-N,N-(C₁-C₆)-alkylamino, N,N-(C₃-C₈)Alkandiylamino, wie z. B. Pyrollidino, Piperidino, N-(C₆-C₁₂)-Arylamino, N(C₆-C₁₂)-Aryl-N-(C₁-C₆)-alkylamino, N-(C₇-C₁₁)-Aralkylamino, N-(C₇-C₁₁)-Aralkyl-N-(C₁-C₁₀)-alkylamino, (C₁-C₆)Alkanoylamino, (C₃-C₈)-Cycloalkanoylamino, (C₁-C₆)-Hydroxyalkanoylamino, (C₁-C₆)-Alkoxy-(C₁-C₆)-alkanoylamino, (C₆-C₁₂)-Arylcarbonylamino, (C₇-C₁₁)-Aralkylcarbonylamino,
oder
- F: einen unsubstituierten oder substituierten (C₆-C₁₂)-Arylrest, (C₇-C₁₁)Aralkylrest oder Heteroarylrest, wobei der genannte Aryl- und Heteroarylrest insbesondere Phenyl, Naphthyl, Thienyl, Furyl, Pyrrolyl oder Pyridin bedeutet, und der weiterhin 1,2 oder 3 gleiche oder verschiedene Substituenten aus der Reihe Hydroxy, Halogen, Cyano, Nitro, (C₁-C₆)-Alkyl, (C₁-C₆)-Alkoxy, Carboxyl, Trifluormethyl, (C₁-C₆)-Hydroxyalkyl, -O-[CH₂-]ₓC_{f}H_{(2f+1-g)}F_{g}, (C₁-C₆)-Alkylcarbonyl, (C₁-C₆-)-Alkoxycarbonyl, Carbamoyl, N-(C₁-C₄)-Alkylcarbamoyl, N,N-Di-(C₁-C₄)-alkylcarbamoyl, (C₁-C₆)-Akylcarbonyloxy, (C₃-C₈)-Cycloalkyl, Phenyl, Benzyl, Phenoxy, Benzyloxy, Amino, N-(C₁-C₁₀)-Alkylamino, Di-N,N-(C₁-C₁₀)-alkylamino, N,N-(C₃-C₈)-Alkandiylamino, wie z. B. Pyrollidino, Piperidino, (C₁-C₁₀)-Alkanoxylamino, (C₆-C₁₂)-Arylcarbonylamino, (C₇-C₁₁)-Aralkylcarbonylamino,
oder
- G: einen Substituenten der Formeln -OR⁹ oder -N(R⁹)₂ ist, wobei
- R⁹: Wasserstoff, Alkyl oder Alkenyl jeweils mit bis zu 6 C-Atomen, einen (C₆-C₁₂)-Arylrest oder einen Heteroarylrest bedeutet, der 1, 2 oder 3 gleiche oder verschiedene Substituenten aus der Reihe Halogen, Hydroxy, Cyano, Nitro, Carboxyl, Trifluormethyl, (C₁-C₆)-Alkyl, (C₁-C₆)-Hydroxyalkyl, (C₁-C₆)-Alkoxy, -O-[CH₂-]ₓC_{f}H_{(2f+1-g)}F_{g}, (C₁-C₆)-Alkylcarbonyl, (C₁-C₆)-Alkoxycarbonyl, Carbamoyl, N-(C₁-C₄)-Alkylcarbamoyl, N,N-Di-(C₁-C₄)-alkylcarbamoyl, (C₁-C₆)-Alkylcarbonyloxy, (C₃-C₈)-Cycloalkyl, Phenyl, Benzyl, Phenoxy, Benzyloxy, Amino, N-(C₁-C₁₀)-Alkylamino, Di-N,N-(C₁-C₁₀)-alkylamino, N,N-(C₃-C₈)-Alkandiylamino, wie z. B. Pyrollidino, Piperidino, (C₁-C₁₀)-Alkanoylamino, (C₆-C₁₂)-Arylcarbonylamino, (C₇-C₁₁)-Aralkylcarbonylamino.

Hierunter sind Verbindungen der allgemeinen Formel I bevorzugt, in denen
R², R⁵ und R⁴ oder R³, sofern R⁴ oder R³ nicht schon oben definiert sind und nicht Wasserstoff sein sollen, stehen für
- D: Halogen, insbesondere Fluor, Chlor oder Brom, Trifluormethyl, (C₁-C₁₂)-Alkyl, -O-[CH₂-]ₓC_{f}H_{(2f+1-g)}F_{g},-OCF₂Cl, -O-CF₂-CHFCl, (C₁-C₈)-Alkylcarbonyl, (C₃-C₈)-Cycloalkyl, Amino, N-(C₁-C₁₀)-Alkylamino, Di-N,N-(C₁-C₁₀)-Alkylamino, N-(C₆-C₁₂)-Arylamino, N-(C₆-C₁₂)-Aryl-N-(C₁-C₈)-alkylamino, N-(C₇-C₁₁)-Aralkylamino, N-(C₇-C₁₁)-Aralkyl-N-(C₁-C₁₀)-alkylamino bedeutet, wobei die in vorstehenden Substituenten vorhandenen Aryl- und Aralkylreste auch heterocyclischer Natur sein können und/oder, wie auch Alkyl, mit 1 oder 2 gleichen oder verschiedenen Substituenten aus der Reihe Halogen, Trifluormethyl, (C₁-C₆)-Alkyl Hydroxy, (C₁-C₆)-Hydroxyalkyl, (C₁-C₆)-Alkoxy, -O-[CH₂-]ₓC₁H_{(2f+1-g)}, Trifluormethyl, NR'-R substituiert sein können oder
- E: einen Alkyl- oder Alkenylrest mit bis zu 5 C-Atomen bedeutet, welcher gegebenenfalls substituiert ist mit 1 oder 2 gleichen oder verschiedenen Substituenten aus der Reihe Hydroxy, Halogen, Trifluormethyl, (C₁-C₆)-Hydroxyalkyl, (C₁-C₆)Alkoxy, (C₁-C₆)-Alkylcarbonyl, (C₁-C₆)-Alkoxycarbonyl, (C₁-C₆)-Alkylcarbonyloxy, Phenyl, Benzyl, Phenoxy, Benzyloxy, Amino, N-(C₁-C₆)-Alkylamino, Di-N,N-(C₁-C₆)-alkylamino oder mit
einem unsubstituierten oder substituierten (C₆-C₁₂)-Arylrest, (C₇-C₁₁)-Aralkylrest oder Heteroarylrest, der 1 oder 2 gleiche oder verschiedene Substituenten aus der Reihe Hydroxy, Halogen, Trifluormethyl, (C₁-C₆)-Alkyl, (C₁-C₆)-Hydroxyalkyl, (C₁-C₆)-Alkoxy, [CH₂-]ₓC_{f}H_{(2f+1-g)}F_{g}, (C₁-C₆)-Alkylcarbonyl, (C₁-C₆)-Alkoxycarbonyl, Phenyl, Benzyl, Phenoxy, Benzyloxy, Amino, N-(C₁-C₆)-Alkylamino, Di-N,N-(C₁-C₆)-alkylamino, N,N-(C₃-C₈)-Alkandiylamino, wie z. B. Pyrollidino, Piperidino, oder
- F: einen unsubstituierten oder substituierten (C₆-C₁₂)-Arylrest, (C₇-C₁₁)Aralkylrest oder Heteroarylrest, wobei der genannte Aryl- und Heteroarylrest insbesondere Phenyl, Naphthyl, Thienyl, Furyl, Pyrrolyl oder Pyridin bedeutet, und der weiterhin 1,2 oder 3 gleiche oder verschiedene Substituenten aus der Reihe Hydroxy, Halogen, (C₁-C₆)-Alkyl, (C₁-C₆)-Alkoxy, Carboxyl, Trifluormethyl, (C₁-C₆)-Hydroxyalkyl, -O-[CH₂-]ₓC_{f}H_{(2f+1-g)}F_{g}, (C₁-C₆)-Alkylcarbonyl, (C₁-C₆)-Alkoxycarbonyl, Amino, N-(C₁-C₁₀)-Alkylamino, Di-N,N-(C₁-C₁₀)-alkylamino, oder
- G: einen Substituenten der Formeln -OR⁹ oder -N(R⁹)₂ ist, wobei
- R⁹: Wasserstoff oder Alkyl jeweils mit bis zu 6 C-Atomen, einen (C₆-C₁₂)-Arylrest oder einen Heteroarylrest bedeutet, der 1 oder 2 gleiche oder verschiedene Substituenten aus der Reihe Halogen, Trifluormethyl, (C₁-C₆)-Alkyl, (C₁-C₆)-Alkoxy,-O-[CH₂-]ₓC_{f}H_{(2f+1-g)}F_{g}, Amino, N-(C₁-C₁₀)-Alkylamino oder Di-N,N-(C₁-C₁₀)-alkylamino enthält.

Besonders bevorzugt sind Verbindungen der allgemeinen Formel I, in denen
R², R⁵ und R⁴ oder R³, sofern R⁴ oder R³ nicht schon oben definiert sind und nicht Wasserstoff sein sollen, stehen für
- D: Halogen, insbesondere Fluor, Chlor oder Brom, Trifluormethyl, (C₁-C₁₂)-Alkyl, -O-[CH₂-]ₓC_{f}H_{(2f+1-g)}F_{g}, -OCF₂Cl, -O-CF₂-CHFCl, (C₁-C₈)-Alkylcarbonyl, (C₃-C₈)-Cycloalkyl, Amino, N-(C₁-C₁₀)-Alkylamino, Di-N,N-(C₁-C₁₀)-Alkylamino, N-(C₆-C₁₂)-Arylamino, N-(C₆-C₁₂)-Aryl-N-(C₁-C₈)-alkylamino, N-(C₇-C₁₁)-Aralkylamino, N-(C₇-C₁₁)-Aralkyl-N-(C₁-C₁₀)-alkylamino bedeutet, oder:
- E: einen Alkyl- oder Alkenylrest mit bis zu 5 C-Atomen bedeutet, welcher gegebenenfalls substitutiert ist mit 1 oder 2 gleichen oder verschiedenen Substituenten aus der Reihe Hydroxy, Halogen, Trifluormethyl, (C₁-C₆)Alkoxy, (C₁-C₆)-Alkylcarbonyl, (C₁-C₆)-Alkoxycarbonyl, (C1-C₆)-Alkylcarbonyloxy, Phenyl, Phenoxy, Benzyloxy, Amino, N-(C₁-C₆)-Alkylamino, Di-N,N-(C₁-C₆)-alkylamino oder mit einem unsubstituierten oder substituierten (C₆-C₁₂)-Arylrest, (C₇-C₁₁)-Aralkylrest oder Heteroarylrest, der 1 oder 2 gleiche oder verschiedene Substituenten aus der Reihe Hydroxy, Halogen, Trifluormethyl, (C₁-C₆)-Alkyl, (C₁-C₆)-Hydroxyalkyl, (C₁-C₆)-Alkoxy, (C₁-C₆)-Alkylcarbonyl, (C₁-C₆)-Alkoxycarbonyl, oder
- F: einen unsubstituierten oder substituierten (C₆-C₁₂)-Arylrest, (C₇-C₁₁)Aralkylrest, wobei der genannte Arylrest insbesondere Phenyl oder Naphthyl, bedeutet und der weiterhin 1 oder 2 gleiche oder verschiedene Substituenten aus der Reihe Hydroxy, Halogen, (C₁-C₆)-Alkyl, (C₁-C₆)-Alkoxy, Carboxyl, Trifluormethyl, (C₁-C₆)-Hydroxyalkyl, (C₁-C₆)-Alkylcarbonyl, (C₁-C₆)-Alkoxycarbonyl, oder
- G: einen Substituenten der Formeln -OR⁹ oder -N(R⁹)₂ ist, wobei
- R⁹: Wasserstoff oder Alkyl jeweils mit bis zu 6 C-Atomen, einen (C₆-C₁₂)-Arylrest oder einen Heteroarylrest bedeutet, der 1 oder 2 gleiche oder verschiedene Substituenten aus der Reihe Halogen, Trifluormethyl, (C₁-C₆)-Alkyl, (C₁-C₆)-Alkoxy, Amino, N-(C₁-C₁₀)-Alkylamino oder Di-N,N-(C₁-C₁₀)-alkylamino enthält.

Weiterhin betrifft die Erfindung die Verwendung von Verbindungen der allgemeinen Formel I sowie die physiologisch verträglichen Salze zur Herstellung eines Prolin- und Lysinhydroxylase-hemmenden Arzneimittels.

Schließlich betrifft die Erfindung die Verbindungen der allgemeinen Formel I zur Verwendung als Arzneimittel.

Insbesondere betrifft die Erfindung die Verbindung der Formel I zur Anwendung als Fibrosuppressiva und Immunsuppressiva sowie zur Inhibierung der Prolin- und Lysinhydroxylase und der Beeinflussung des Stoffwechsels von Kollagen und kollagenähnlichen Stoffen bzw. der Biosynthese von C1q.

Weiterhin betrifft die Erfindung ein Verfahren zur Herstellung von Verbindungen der allgemeinen Formel I.

Das folgende Schema I gilt für die Herstellung von 2,4-disubstituierten Pyridin-N-oxid-derivaten. Es ist aber grundsätzlich auch zur Verdeutlichung der Herstellung von 2,5-disubstituierten Pyridin-N-oxid-derivaten, sowie für gemischte Derivate (Ester/Amide) anwendbar. 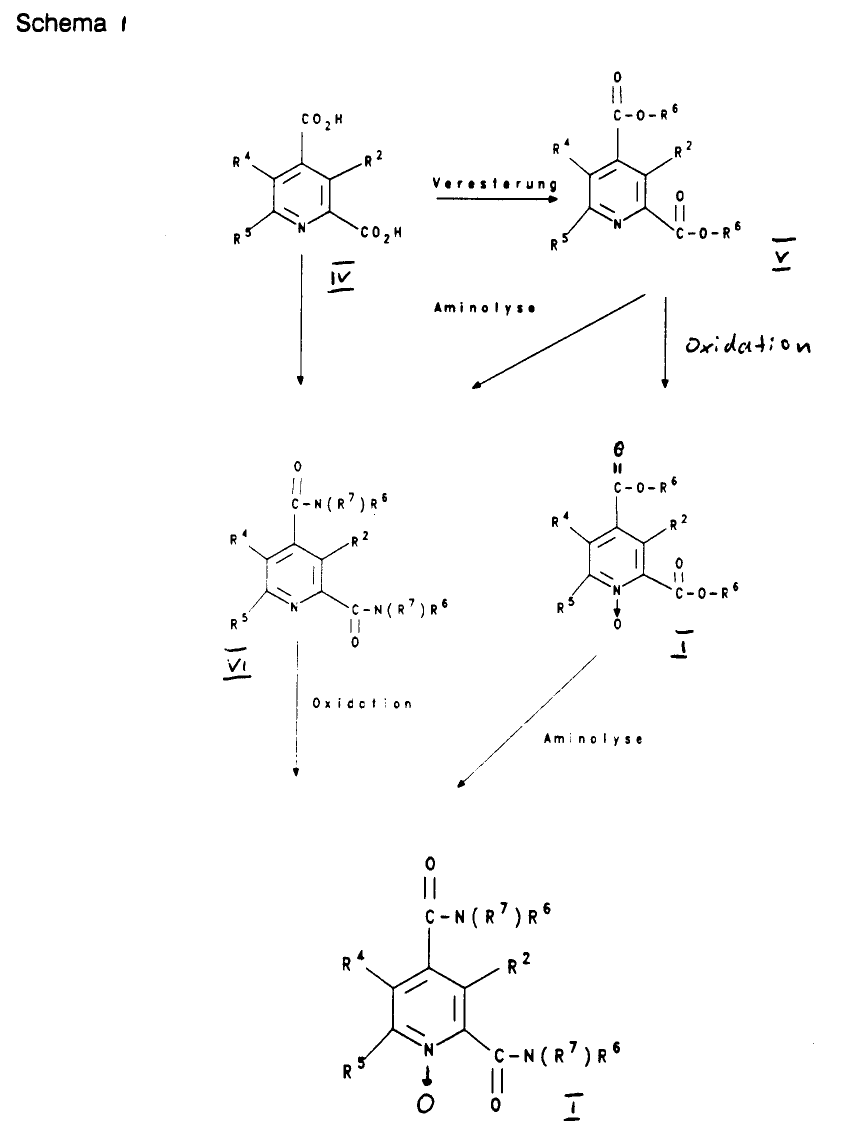
Substituierte Pyridin-2,4- und -2,5-dicarbonsäuren der Formel IV werden z. B. durch Oxidation aus den entsprechenden Dimethylpyridinen VII bzw. VIII oder den entsprechenden Methyl-2-hydroxymethylpyridinen IX erhaften. Die Herstellung der Dimethylpyridine VII bzw. VIII ihrerseits wird in R.J. Ife, J. Med. Chem. 32, 1970-1977 (1989) und der dort zitierten Literatur beschrieben. Das folgende Schema II gibt hierzu einen Anhaltspunkt. Aufgrund dieses Schemas sind dem Fachmann die erfindungsgemäßen substituierten Pyridinderivate zugänglich. Ein besonderes Beispiel ist 2,5-Dimethyl-3,4-dichlorpyridin 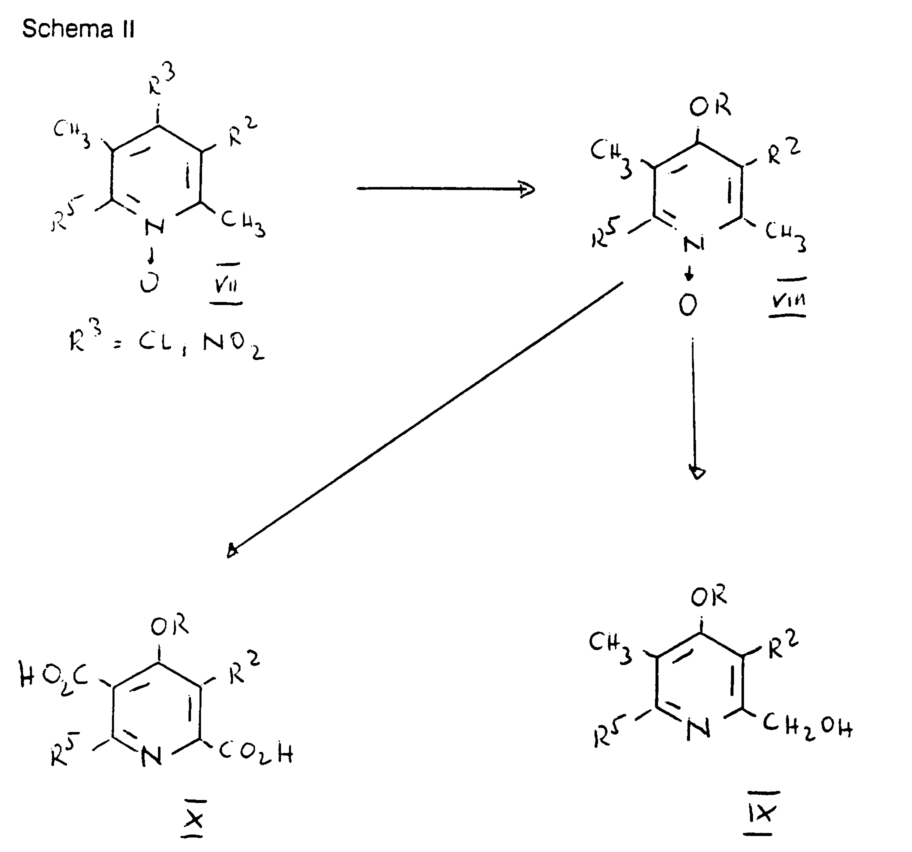
Die Umsetzung von Verbindungen der Formel VII zu Verbindungen der Formel VIII geschieht mit Alkoholen ROH, einer Base in DMF oder Dimethylacetamid bei 20 bis 150° C. Verbindungen des Typs IX werden durch Reaktionen von VIII mit Acetanhydrid/Essigsäure bei 120° C und nachfolgender Umsetzung mit NaOH/Methanol bei 25° C hergestellt. Die Oxidation zu Verbindung X mit Natriumpermanganat in Wasser bei 70 bis 100° C kann ausgehend von VIII oder IX erfolgen. Die Dicarbonsäuren X können nach Schema I entweder verestert und zu den erfindungsgemäßen N-oxiden I oxidiert werden oder - ebenso nach Schema I - in Amide überführt und oxidiert werden.

Die Herstellung der Verbindungen des Types IV wird von den folgenden Literaturstellen beschrieben. Hierbei handelt es sich um die Einführung der Substituenten R², R⁴, R⁵ in die 2,4-bzw. 2,5-Pyridine-dicarbonsäure.

Beginnend mit der Verbindung der Formel IV kann das Amid VI entweder direkt aus IV oder über den Ester V hergestellt werden.

Die Reaktion von V oder VI zu den Amiden I oder Estern I erfolgt analog zu den Bedingungen aus der P 40 20 570.3.

Hierzu wird ein Oxidationsmittel wie z. B. Wasserstoffperoxid oder Persäuren wie Peressigsäure, Perfluoressigsäure, Perbenzoesäure oder Metachlorperbenzoesäure in Lösungsmitteln wie chlorierte Kohlenstoffe, wie z. B. Methylenchlorid, Chloroform, Tri- oder Tetrachlorethylen, Benzol oder Toluol zu den oxidierenden Pyridinverbindungen, die ebenfalls in den obengenannten Lösungsmitteln gelöst sein können, gegeben und bei einer Temperatur zwischen -30 und +40°C bevorzugt 0 und +25°C zwischen 30 Minuten und 3 Tagen gerührt. Die Beendigung der Reaktion läßt sich beispielsweise mittels Dünnschichtchromatographie bestimmen. Vorzugsweise lassen sich die erfindungsgemäßen Verbindungen herstellen, in dem man das Pyridinderivat und das Oxidationsmittel in äquimolaren Mengen oder bis zu einem etwa 5-fachen Überschuß an Oxidationsmittel einsetzt.

Gegebenenfalls kann auch ein Überschuß an Persäure beseitigt werden, in dem man beispielsweise gasförmigen Ammoniak in die Reaktionslösung einleitet und den entstehenden Niederschlag durch Filtration von der Reaktionslösung abtrennt.

Gegebenenfalls kann die Aufarbeitung der Produkte beispielsweise durch Extraktion oder durch Chromatographie z. B. über Kieselgel erfolgen. Das isolierte Produkt kann umkristallisiert werden.

Eine allgemeine Vorschrift dieser Oxidationsmethode ist auch beispielsweise in "E. Lingsberg, Pyridine and its Derivtives, Interscience Publishers, New York, 1961, Part 2, 93" beschrieben.

Die Oxidation mit Wasserstoffperoxid ist beispielsweise in "E. Ochiai, J. Org. Chem. 18, 534 (1953)" beschrieben.

Die Bedingungen für eine Aminolyse von Ester I zum Amid I entsprechen denen, die bei der Reaktion von V zu VI angewandt werden.

Im übrigen sind die Herstellung und die Aminolyse als solches bekannte Reaktionstypen: Die mit obigen Reaktionen hergestellten Verbindungen der allgemeinen Formel I sind aber neu.

Die Verfahrensbedingungen können im Detail der deutschen Patentanmeldungen P 38 26 471.4, 38 28 140.6, 39 24 093.2, 41 01 002.3 sowie den DE-A-37 03 959, 37 03 962 und 37 03 963 entnommen werden.

Die erfindungsgemäßen Verbindungen der Formel I besitzen wertvolle pharmakologische Eigenschaften und zeigen insbesondere Wirksamkeit als Hemmer der Prolin- und Lysinhydroxylase, als Fibrosuppressivum, Immunsuppressivum und Antiatherosklerotikum.

Die antifibrotische Wirkung kann im Modell der Tetrachlorkohlenstoff-induzierten Leberfibrose bestimmt werden. Dazu werden Ratten mit CCl₄ (1 ml/kg) - gelöst in Olivenöl - zweimal wöchentlich behandelt. Die Prüfsubstanz wird täglich, gegebenenfalls sogar zweimal täglich per os oder intraperitoneal - gelöst in einem geeigneten verträglichen Lösungsmittel - verabreicht. Das Ausmaß der Leberfibrose wird histologisch bestimmt und der Anteil Kollagen in der Leber per Hydroxyprolinbestimmung - wie bei Kivirikko et al. (Anal. Biochem. 19, 249 f. (1967)) beschrieben- analysiert. Die Aktivität der Fibrogenese kann durch radioimmunologische Bestimmung von Kollagenfragmenten und Prokollagenpeptiden im Serum bestimmt werden. Die erfindungsgemäßen Verbindungen sind in diesem Modell in Konzentration 1 - 100 mg/kg wirksam.

Die Aktivität der Fibrogenese kann durch radioimmunologische Bestimmung des N-terminalen Propeptids des Kollagens Typ-III oder der N- bzw. C-terminalen Quervernetzungsdomäne des Kollagens-Typ-IV (7s-Kollagen bzw. Typ-IV-Kollagen-NC₁) im Serum bestimmt werden.

Zu diesem Zweck wurden die Hydroxyprolin-, Prokollagen-III-Peptid-, 7s-Kollagen- und Typ-IV-Kollagen-NC-Konzentrationen in der Leber von
a) unbehandelten Ratten (Kontrolle)
b) Ratten, denen Tetrachlorkohlenstoff verabreicht wurde (CCl₄-Kontrolle)
c) Ratten, denen zunächst CCl₄ und anschließend eine erfindungsgemäße Verbindung verabreicht wurde
gemessen (diese Testmethode wird beschrieben von Rouiller, C., experimental toxic injury of the liver; in The Liver, C. Rouiller, Vol. 2, 5. 335-476, New York, Academic Press, 1964).

Ein anderes Modell zur Evaluierung der antibiotischen Wirkung ist das der Bleomycin-induzierten Lungenfibrose wie bei Kelley et al. (j. Lab. Clin. Med. 96, 954, (1980) beschrieben. Für die Evaluierung der Wirkung der erfindungsgemäßen Verbindungen der Granulationsgewebe kann das Modell des Wattebauschgranuloms, wie bei Meier et al., Experimentia 6, 469 (1950) beschrieben, herangezogen werden.

Die Verbindungen der Formel I können als Medikamente in Form pharmazeutischer Präparate Verwendung finden, welche sie gegebenenfalls zusammen mit verträglichen pharmazeutischen Trägern enthalten. Die Verbindungen können als Heilmittel, z. B. in Form pharmazeutischer Präparate Verwendung finden, welche diese Verbindungen in Mischung mit einem für die enterale, perkutane oder parenterale Applikation geeigneten pharmazeutischen, organischen oder anorganischen Träger, wie z. B. Wasser, Gummi arabicum, Gelatine, Milchzucker, Stärke, Magnesiumstearat, Talk, pflanzliche Öle, Polyalkylenglykole, Vaseline usw. enthalten.

Sie können zu diesem Zweck oral in Dosen von 0,1 - 25 mg/kg/Tag, vorzugsweise 1 - 5 mg/kg/Tag oder parenteral in Dosen von 0,01 - 5 mg/kg/Tag, vorzugsweise 0,01 - 2,5 mg/kg/Tag, insbesondere 0,5 - 1,0 mg/kg/Tag, appliziert werden. Die Dosierung kann in schweren Fällen auch erhöht werden. In vielen Fällen genügen jedoch auch geringere Dosen. Diese Angaben beziehen sich auf einen Erwachsenen von etwa 75 kg Gewicht.

Die Erfindung umfaßt weiterhin die Verwendung der erfindungsgemäßen Verbindungen bei der Herstellung von Arzneimitteln, die zur Behandlung und Prophylaxe der vorstehend genannten Stoffwechselstörungen eingesetzt werden.

Ein weiterer Gegenstand der Erfindung sind Arzneimitel, die ein oder mehrere erfindungsgemäße Verbindungen der Formel I und/oder deren physiologisch verträgliche Salze enthalten.

Die Arzneimittel werden nach an sich bekannten, dem Fachmann geläufigen Verfahren hergestellt. Als Arzneimittel werden die erfindungsgemäßen pharmakologisch wirksamen Verbindungen (= Wirkstoff) entweder als solche oder vorzugsweise in Kombination mit geeigneten pharmazeutischen Hilfs- oder Trägerstoffen in Form von Tabletten, Dragees, Kapseln, Suppositorien, Emulsionen, Suspensionen oder Lösungen eingesetzt, wobei der Wirkstoffgehalt bis etwa 95 %, vorteilhafterweise zwischen 10 und 75 % beträgt.

Geeignete Hilfs- bzw. Trägestoffe für die gewünschte Arzneimittelformulierung sind beispielsweise neben Lösemitteln, Gelbildner, Suppositoriengrundlagen, Tabletten-Hilfsstoffen und anderen Wirkstoffträgern auch Antioxidantien, Dispergiermittel, Emulgatoren, Entschäumer, Geschmachskorrigentien, Konservierungsmittel, Lösungsvermittler oder Farbstoffe.

Die folgenden Beispiele sollen die Erfindung erläutern.

### Beispiel 1

### 5-Brom-pyridin-1-oxid-2,4-dicarbonsäure- bis [(3-methoxypropyl)amid]

a) 5-Brom-2,4-dimethylpyridin
   Zu 28,9 ml 2,4-Dimethylpyridin wird unter Eiskühlung und Rühren 150 ml 65 % Oleum so zugetropft, daß die Temperatur 35°C nicht übersteigt. Hat sich die Lösung homogenisiert, wird langsam unter Rühren 6,42 ml Brom zugetropft. Es wird bei 80°C 3 ½ Stunden gerührt. Nach dem Abkühlen läßt man vorsichtig auf 1 kg Eis tropfen, neutralisiert mit festem Na₂CO₃ und extrahiert 3 mal mit je 300 ml Ether. Die organische Schicht wird abgetrennt und über Magnesiumsulfat getrocknet. Nach Entfernen des Lösungsmittels durch Destillation im Vakuum erhält man 34,6 g blaßgelbes Öl, welches aus den Isomeren 5-Brom-2,4-dimethylpyridin und 3-Brom-2,4-dimethylpyridin besteht. Die Isomere werden durch Säulenchromatographie an Siliziumdioxidgel getrennt und man erhält 10 g 5-Brom-2,4-dimethylpyridin als farbslose Flüssigkeit (13,0 g 3-Brom-2,4-dimethylpyridin).
   Ausbeute: 22 %.
b) 5-Brom-pyridin-2,4-dicarbonsäure
   4 g 5-Brom-2,4-dimethylpyridin aus Beispiel 1 werden in 200 ml Wasser und 2,4 g KOH auf 70-80°C erhitzt. Dann wird portionsweise die Hälfte von 12,74 g Kaliumpermanganat eingetragen. Die Lösung wird zum Sieden erhitzt und der Rest des Kaliumpermanganats zugegeben. Man läßt 20 h bei 70-80°C rühren, saugt dann heiß ab und wäscht den Niederschlag 4 mal mit 50 ml-Portionen heißem Wasser. Die vereinigten Filtrate werden im Vakuum auf 100 ml eingeengt. Man stellt die Lösung mit konzentrierter Salzsäure auf pH 1 ein und läßt die Lösung für 20 Stunden bie 0°C stehen. Der kristalline Feststoff wird abgesaugt und bei 100°C im Vakuum getrocknet. Die Ausbeute beträgt 2,9 g.
   Fp. 261-263°C.
c) 5-Brom-pyridin-2,4-dicarbonsäure-bis [3-methoxypropyl)amid]
   2,46 (10 mmol) 5-Brom-pyridin-2,4-dicarbonsäure werden in 70 ml Toluol und 0,2 ml Dimethylformamid suspendiert und mit 1,45 ml (20 mmol) Thionylchlorid versetzt. Das Reaktionsgemisch wird 5 h auf 110°c erhitzt, wobei eine klare Lösung entsteht.
   Dann läßt man auf 0°C abkühlen und tropft eine Lösung von 2,05 ml (20 mmol) 3-Methoxypropylamin und 2,8 ml Triethylamin in 10 ml Toluol zu.
   Man läßt 12 Stunden bei 20°C stehen, engt im Vakuum ein, versetzt mit Wasser, extrahiert mit Dichlormethan, trocknet die organische Phase, befreit vom Lösungsmittel und erhält die Substanz als farbloses Öl, Ausbeute 2,2 g
   Summenformel: C₁₅ H₂₂ Br N₃ O₄ (388,26)
   MS: m/e = 389 (M + H⁺)
d) Die vorstehende Verbindung wird in 80 ml Dichlormethan gelöst und bei 10°C portionsweise mit 5 g meta-Chlor-perbenzoesäure versetzt. Nach 2 Stunden gibt man weitere 5 g Persäure hinzu, erwärmt 3 Stunden auf 42°C, gibt nochmals 5 g Persäure hinzu und erwärmt 5 Stunden auf 42°C.
   Überschüssige Persäure und 3-Chlorbenzolsäure werden durch Einleiten von Ammoniak-Gas ausgefällt und abgesaugt. Die Lösung wird eingeengt und der Rückstand mit Ethylacetat/Methanol 10: 1 an Kieselgel chromatographiert. Man erhält die Titelverbindung als farblose Kristalle. Fp. 94° C.
   Summenformel: C₁₅ H₂₂ Br N₃ O₅ (404,26)
   MS: m/e = 405 (M + H⁺)

### Beispiel 2

### Pyridin-1-oxid-2,4-dicarbonsäuredimethylester

7,0 g (35.8 mmol) Pyridin-2,4-dicarbonsäure-dimethylester werden in 200 ml Dichlormethan gelöst und bei 10°C mit 6,2 g (36 mmol) 3-Chlorbenzoesäure versetzt. Nach 2 Stunden gibt man weitere 6.2 g Persäure, läßt über Nacht stehen, gibt weitere 3.1 g Persäure zu, erwärmt 5 h auf 40°C (DC-Kontrolle). Überschüssige Persäure und 3-Chlorbenzoesäure werden durch 3-maliges Einleiten von Ammoniakgas ausgefällt und abfiltriert. Die Lösung wird eingeengt und der Rückstand aus Toluol kristallisiert, 4,5 g.
Fp. 132°C; R_{f} (SiO₂, Ethylacetat) = 0.29

### Beispiel 3

### Pyridin-1-oxid-2-carbonsäure-methylester-4-carbonsäure-(2-benzyloxyethyl)amid und Pyrridin-1-oxid-2-carbonsäure-(2-benzyloxyalkyl)amid-4-carbonsäure-methylester

3,1 g (15 mmol) der Titelverbindung aus Beispiel 2 werden in 50 ml N,N-Dimethyl-acetamid mit 22,8 g (150 mmol) 2-Benzyloxyethylamin (hergestellt aus 2-Aminoethanol, Natrium und Benzylchlorid) 1 Stunde auf 100°C erhitzt. Man läßt Abkühlen, engt ein, versetzt mit Wasser, extrahiert mit Dichlormethan, engt ein und bringt den öligen Rückstand (4,8 g) mit Ethylacetat zur Kristallisation.
Fp. 76-78°C (farblose Kristalle)
Summenformel: C₁₇ H₁₈ N₂ O₅ (330)
MS: m/e = 331 (M + H⁺)
Das entsprechende Diamid konnte nach 10 h bei 140°C erhalten werden.

### Beispiel 4

### 4-Methoxy-pyridin-1-oxid-2,5-dicarbonsäure-bis-[(2-hydroxyethyl)amid]

a) 2-Hydroxymethyl-4-methoxy-5-methyl-pyridin
   5,4 g (35 mmol) 4-Methoxy-2,5-dimethyl-pyridin-N-oxid (Fp. 102-104, Diisopropylether; hergestellt aus 2,5-Dimethyl-pyridin-N-oxid und Natriummethylat in Methanol) werden in 30 ml Eisessig gelöst und bei 80°C tropfenweise mit 40 ml. Acetanhydrid versetzt.
   Man erhitzt 90 min auf 115°C, kühlt auf 80°C ab, tropft 75 ml Methanol zu, engt ein, nimmt den Rückstand in Methanol auf, tropft 200 ml 1,5 H methanolische Natronlauge zu, versetzt mit Wasser, extrahiert mit Dichlormethan, trocknet, engt ein und bringt mit Cyclohexan zur Kristallisation, Ausbeute: 4,4 g; Fp. 92-94° C. Fp.92-94°C.
b) 4-Methoxy-pyridin-2,5-dicarbonsäure
   2,3 g (15 mmol) der Verbindung aus 4a) werden in einer Lösung von 1g KOH in 75 ml Wasser suspendiert und bei 70°C portionsweise mit 7,2 g (45 mmol) KMnO₄ versetzt. Man läßt noch 1 Stunde bei 80°C, wäscht den abgesaugten Braunstein mit wäßrigem Wasser, engt das Filtrat ein und bringt mit Salzsäure auf je pH1; Ausbeute 1,45 g; Fp 231°C (Zersetzung).
c) 4-Methoxy-pyridin-1-oxid-2,5-dicarbonsäure-dimethylester
   1,4 g der Verbindung aus 4b) werden in 150 ml Methanol und 2 g Schwefelsäure (98 %) 15 Stunden zum Sieden erhitzt. Man läßt abkühlen, versetzt unter Kühlen mit gesättigter Ammoniumchlorid-Lösung, extrahiert mit Dichlormethan, engt ein und erhält analog Beispiel 2 durch Umsetzung des Rohprodukts mit 3-Chlorbenzolsäure 0,8 g 4-Methoxy-pyridin-2,5-dicarbonsäure-dimethylester-N-oxid, Fₚ 134-136° C.
d) Die Titelverbindung erhält man aus der vorstehenden Verbindung des Beispiels 4c) durch Reaktion mit überschüssigem 2-Aminoethanol, farblose Kristalle, Fp. 124-127°C (aus Ethylacetat).
   Analog konnten aus der Verbindung des Beispiels 4c) durch Reaktion mit den entsprechenden Aminen folgende Verbindungen erhalten werden:
   4-Methoxy-pyridin-2,5-dicarbonsäure-bis [(2-methoxyethyl)amid]-N-oxid
   4-Methoxy-pyridin-2,5-dicarbonsäure-bis [(3-hydroxypropyl)amid]-N-oxid
   4-Methoxy-pyridin-2,5-dicarbonsäure-bis [(3-methoxypropyl)amid]-N-oxid
   4-Methoxy-pyridin-2,5-dicarbonsäure-bis [(2-benzyloxyethyl)amid]-N-oxid

### Beispiel 5

### Pyridin-1-oxid-2,4-dicarbonsäure-bis-N,N'-[2-(4-methylbenzoyloxy)-ethyl-amid

a) Pyridin-2,4-dicarbonsäure-bis-N,N'-[2-(4-methylbenzoyloxy)-ethyl]-amid
b) 0,7 g (2,5 mM) Pyridin-2,4-dicarbonsäure-bis-N,N'-(3-hydroxyethyl)-amid werden in 100 ml Dichlormethan mit 0,2 g 4-N,N-Dimethylaminopyridin, 0,8 ml (6 mM) Triethylamin und sodann tropfenweise mit 0,6 ml (5 mM) 4-Methylbenzoylchlorid versetzt. Nach 1 Stunde wird eingeengt, dann mit Wasser aufgenommen.
   Nach 1 Stunde wird zweimal mit Wasser ausgeschüttelt und die organische Phase eingeengt. Das Rohprodukt wird mit Ethylacetat an Kieselgel chromatographiert.
   Farbloses Kristallpulver, Fp. 165-166°C
   Summenformel: C₂₇H₂₇N₃O₆(489)
   MS: m/e = 490 (M + H⁺).
c) Zu einer Lösung von 0,25 g der vorstehenden Verbindung in 5 ml 96-prozentiger Ameisensäure werden 0,31 ml 30-prozentiges H₂O₂ getropft und das Gemisch 4 Stunden auf 80°C erwärmt. Anschließend wird i.Vak. eingedampft und der kristalline Rückstand mit warmem Methanol verrührt und abgesaugt. Man erhält 0.18 g Pyridin-2,4-dicarbonsäure-N,N'-bis-[2-(4-methylbenzoyloxy)-ethyl]-amid als farbloses Kristallpulver, Fp. 186-188°C.
   MS: m/e = 506 (M⁺ + H⁺).

### Beispiel 6

### Pyridin-1-oxid-2,4-dicarbonsäure-N,N'-bis-[(2-benzoyloxyethyl)-amid]

a) Pyridin-2,4-dicarbonsäure-bis-N,N'-[(2-benzoyloxyethyl)amid]
   analog Beispiel 5a)
   farblose Nadeln, Fp. 139-140°C
   Summenformel: C₂₅H₂₃N₃O₆(461)
   MS: m/e = 462(M⁺+H⁺).
b) Zu einer Lösung von 0.46 g Pyridin-2,4-dicarbonsäure-N,N'-bis-[(2-benzoyloxy)ethyl]-amid (Beispiel 6a)) in 5 ml 96-prozentiger Ameisensäure werden 0,31 ml 30-prozentiges H₂O₂ getropft und das Gemisch unter Rühren 4 Stunden auf 80°C erwärmt. Nach Zugabe von weiteren 0.2 ml H₂O₂ wird nochmals 2 Stunden bei dieser Temperatur gerührt, anschließend i.Vak. eingedampft und der Rückstand durch Chromatographie an Kieselgel mit Ethylacetat als Eluens gereinigt. Man erhält 0,37 g Pyridin-1-oxid-2,4-dicarbonsäure-N,N-bis-[(2.benzoyloxyethyl)amid] farblose Kristalle, Fp. 159-160°C (Methanol).
   MS: m/e = 478(M⁺+H⁺).

## Patentansprüche

1. Substituierte Pyridin-N-oxide der allgemeinen Formel I, 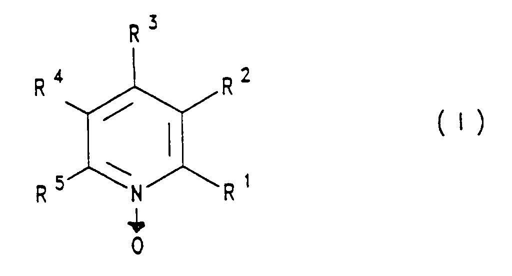 worin
R¹ und R³ oder R⁴ -C(O)-X-R⁶ bedeuten, wobei
X O oder -N-(R⁷)- und
R⁶ und R⁷ gleich oder verschieden sind und
A einen verzweigten oder unverzweigten, aliphatischen oder cycloaliphatischen (C₁-C₁₂)-Alkylrest, (C₁-C₁₂)-Alkenylrest oder einen (C₁-C₁₂)-Alkinylrest bedeutet,
der unsubstituiert oder einfach oder mehrfach, vorzugsweise einfach oder zweifach substituiert ist mit Halogen, insbesondere Fluor, Chlor, Brom, Hydroxy, Cyano, Carboxyl, (C₁-C₈)-Alkoxy, (C₁-C₈)-Alkoxycarbonyl, (C₁-C₈)-Alkoxycarbonyloxy, (C₁-C₈)-Alkoxy-(C₁-C₈)-alkoxycarbonyloxy, (C₆-C₁₂)-Aryloxycarbonyloxy, (C₇-C₁₁)-Aralkyloxycarbonyloxy, (C₇-C₁₁)-Aralkylcarbonyloxy, Cinnamoyl, Cinnamoyloxy, (C₆-C₁₂)-Arylcarbonyloxy, (C₃-C₈)-Alkenylcarbonyloxy, (C₃-C₈)-Alkinylcarbonyloxy, (C₃-C₈)-Cycloalkylcarbonyloxy, (C₁-C₁₂)-Alkoxy-(C₁-C₁₂)-alkoxy, (C₁-C₁₂)-Alkoxy-amino, (C₁-C₁₂)-Alkoxy-N (C₁-C₆)-alkylamino, (C₁-C₁₂)-Alkoxy-N,N (C₁-C₆)-dialkylamino, Carbamoyloxy, N-(C₁-C₈)-Alkylcarbamoyloxy, N,N-Di-(C₁-C₈)-alkylcarbamoyl, N-(C₃-C₈)-Cycloalkylcarbamoyl, N-(C₆-C₁₂)-Arylamino, N-(C₇-C₁₁)-Aralkylamino, N-Alkyl-Aralkylamino, N-Alkyl-Arylamino, (C₃-C₈)-Cycloalkanoylamino, (C₁-C₈)-Alkanoylamino, (C₆-C₁₂)-Aroylamino, (C₇-C₁₁)-Aralkanoylamino, (C₁-C₆)-Alkanoyl-(C₁-C₈-)alkylamino, (C₃-C₈)-Cycloalkanoyl-(C₁-C₈)-alkylamino,(C₆-C₁₂)-Aroyl-(C₁-C₈)-alkylamino, (C₇-C₁₁)-Aralkanoyl-(C₁-C₈)-alkylamino,(C₁-C₈)-Alkylmercapto, (C₁-C₈)-Alkylsulfinyl, (C₁-C₈)-Alkylsulfonyl, (C₁-C₈)-Alkylcarbonyl,(C₃-C₈)-Cycloalkylcarbonyl, Nitro, Trifluormethyl, Phenylmercapto, Phenylsulfonyl, Phenylsulfinyl, Sulfamoyl, N-(C₁-C₆)-Alkylsulfamoyl, N,N-Di(C₁-C₆)-alkylsulfamoyl, (C₁-C₈)-Alkyl-sulfonamido, Arylsulfonamido, wobei die in vorstehenden Substituenten vorhandenen Aryl- und Aralkylreste auch heterocyclischer Natur sein können und/oder, wie auch Alkyl, mit 1, 2, 3, 4 oder 5 gleichen oder verschiedenen Substituenten aus der Reihe Halogen, Cyano, Nitro, Trifluormethyl, (C₁-C₆)-Alkyl-Hydroxy, (C₁-C₆)-Hydroxyalkyl, (C₁-C₆)-Alkoxy, -O-[CH₂-]ₓC_{f}H_{(2f+1-g})F_{g}, -OCF₂Cl, -O-CF₂-CHFCl, Trifluormethyl, (C₁-C₆)-Alkylmercapto, (C₁-C₆)-Alkylsulfinyl, (C₁-C₆)-Alkylsulfonyl, (C₁-C₆)-Alkylcarbonyl, (C₁-C₆)-Alkoxycarbonyl, Carbamoyl, N-(C₁-C₄)-Alkylcarbamoyl, N,N-Di-(C₁-C₄)-alkylcarbamoyl, (C₁-C₆)-Alkylcarbonyloxy, (C₃-C₈)-Cycloalkyl, Phenyl, Benzyl, Phenoxy, Benzyloxy, NR'-R'', Phenylmercapto, Phenylsulfonyl, Phenylsulfinyl, Sulfamoyl, N-(C₁-C₄)-Alkylsulfamoyl oder N,N-Di-(C₁-C₄)-alkylsulfamoyl, insbesondere bis zu mit 3 der vorstehend genannten gleichen oder verschiedenen Substituenten substituiert sind und eine CH₂-Gruppe der Alkylkette gegebenenfalls durch O, S, SO, SO₂ oder NR' ersetzt ist, oder mit
einem unsubstituierten oder substituierten (C₆-C₁₂)-Arylrest oder Heteroarylrest, der 1, 2, 3, 4 oder 5 gleiche oder verschiedene Substituenten aus der Reihe Halogen, Nitro, Cyano, Carboxyl, Hydroxy, Trifluormethyl, (C₁-C₆)-Hydroxyalkyl,-O-[CH₂-]ₓ C_{f}H_{(2f+1-g)}F_{g ,}-OCF₂Cl, -OCF₂-CHFCl, (C₁-C₆)-Alkylmercapto, (C₁-C₆)-Alkylsulfinyl, (C₁-C₆)-Alkylsulfonyl, (C₁-C₈)-Alkoxy, (C₁-C₈)-Alkyl, (C₁-C₆)-Alkylcarbonyl, (C₁-C₆)-Alkoxycarbonyl, Carbamoyl, N-(C₁-C₄)-Alkylcarbamoyl, N,N-Di-(C₁-C₄)-alkylcarbamoyl, (C₁-C₆)-Alkylcarbonyloxy, (C₃-C₈)-Cycloalkyl, Phenyl, Benzyl, Phenoxy, Benzyloxy, NR'-R'', Phenylmercapto, Phenylsulfonyl, Phenylsulfinyl, Sulfamoyl, N-(C₁-C₄)-Alkylsulfamoyl, N,N-Di-(C₁-C₄)-alkylsulfamoyl, (C₁-C₈)-Alkoxycarbonyloxy, (C₁-C₈)-Alkoxy-(C₁-C₈)-alkoxycarbonyloxy, (C₆-C₁₂)-Aryloxycarbonyloxy, (C₇-C₁₁)-Aralkyloxycarbonyloxy, (C₇-C₁₁)-Aralkylcarbonyloxy, Cinnamoyl, Cinnamoyloxy, (C₆-C₁₂)-Arylcarbonyloxy, (C₃-C₈)-Alkenylcarbonyloxy, (C₃-C₈)-Alkinylcarbonyloxy, (C₃-C₈)-Cycloalkylcarbonyloxy, (C₁-C₁₂)-Alkoxy-(C₁-C₁₂)-alkoxy, (C₁-C₁₂)-Alkoxy-amino, (C₁-C₁₂)-Alkoxy-N-(C₁-C₆)-alkylamino, (C₁-C₁₂)-Alkoxy-N,N(C₁-C₆)-dialkylamino, Carbamoyloxy, N-(C₁-C₈)-Alkylcarbamoyloxy, N,N-Di-(C₁-C₈)-alkylcarbamoyl, N-(C₃-C₈)-Cycloalkylcarbamoyl, N-(C₆-C₁₂)-Arylamino, N-(C₇-C₁₁)-Aralkylamino, N-Alkyl-Aralkylamino, N-Alkyl-Arylamino, (C₃-C₈)-Cycloalkanoylamino, (C₁-C₈)-Alkanoylamino, (C₆-C₁₂)-Aroylamino, (C₇-C₁₁)-Aralkanoylamino, (C₁-C₈)-Alkanoyl-(C₁-C₈)-alkylamino, (C₃-C₈)-Cycloalkanoyl-(C₁-C₈)-alkylamino,(C₆-C₁₂)-Aroyl-(C₁-C₈)-alkylamino, (C₇-C₁₁)-Aralkanoyl-(C₁-C₈)-alkylamino,(C₁-C₈)-Alkylmercapto, (C₁-C₈)-Alkylsulfinyl, (C₁-C₈)-Alkylsulfonyl, (C₁-C₈)-Alkylcarbonyl,(C₃-C₆)-Cycloalkylcarbonyl, Nitro, Trifluormethyl, Phenylmercapto, Phenylsulfonyl, Phenylsulfinyl, Sulfamoyl, N-(C₁-C₆)-Alkylsulfamoyl, N,N-Di-(C₁-C₆)-alkylsulfamoyl, (C₁-C₈)-Alkyl-sulfonamido und Arylsulfonamido trägt, wobei die in vorstehenden Substituenten vorhandenen Aryl- und Aralkylreste auch heterocyclischer Natur sein können und/oder, wie auch Alkyl, mit 1, 2, 3, 4 oder 5 gleichen oder verschiedenen Substituenten aus der Reihe Halogen, Cyano, Nitro, Trifluormethyl, (C₁-C₆)-Alkyl, Hydroxy, (C₁-C₆)-Hydroxyalkyl, (C₁-C₆)-Alkoxy, substituiert sein können, oder mit
einem unsubstituierten oder substituierten (C₆-C₁₂)-Aryloxyrest, (C₇-C₁₁)-Aralkyloxyrest oder Heteroaryloxyrest, der 1, 2, 3, 4 oder 5 gleiche oder verschiedene Substituenten aus der Reihe Hydroxy, Halogen, Cyano, Nitro, Trifluormethyl, (C₁-C₆) Alkyl, (C₁-C₆)-Hydroxyalkyl, (C₁-C₆)Alkoxy, [CH₂-]ₓC_{f}H_{(2f+1-g)}F_{g}, -OCF₂-CHFCl, (C₁-C₆)-Alkylmercapto, (C₁-C₆)-Alkylsulfinyl, (C₁-C₆)-Alkylsulfonyl, (C₁-C₆)-Alkylcarbonyl, (C₁-C₆)-Alkoxycarbonyl, Carbamoyl, N-(C₁-C₄)-Alkylcarbamoyl, N,N-Di-(C₁-C₄)-alkylcarbamoyl, (C₁-C₆)-Alkylcarbonyloxy, (C₃-C₈)-Cycloalkyl, Carboxyl, Phenyl, Benzyl, Phenoxy, Benzyloxy, NR'-R'', Phenylmercapto, Phenylsulfonyl, Phenylsulfinyl, Sulfamoyl, N-(C₁-C₄)-Alkylsulfamoyl, N,N-Di-(C₁-C₄)-alkylsulfamoyl, Aminoalkyl, N-(C₁-C₈)-alkylamino-(C₁-C₁₂)-alkyl oder N-Di-(C₁-C₈)-alkylamino-(C₁-C₁₂)-alkyl trägt, gegebenenfalls bis zu mit 3 der vorstehend genannten gleichen oder verschiedenen Substituenten substituiert ist und eine CH₂-Gruppe der Alkylkette gegebenenfalls durch O, S, SO, SO₂ oder NR' ersetzt ist, oder mit
einem Rest der allgemeinen Formel II
- O - R⁸ (II),
worin
R⁸ eine über ihren Acylrest gebundene Aminosäure, ihr Derivat oder eine Alkoholschutzgruppe bedeutet,
B einen unsubstituierten oder substituierten (C₆-C₁₂)Arylrest, (C₇-C₁₁)Aralkylrest oder einen Heteroarylrest bedeutet, der einfach oder mehrfach, vorzugsweise ein- oder zweifach substituiert ist mit
Hydroxy, Halogen, Cyano, Carboxyl, Amino, (C₁-C₈)-Alkyl, (C₁-C₈)-Alkoxy, (C₁-C₈)-Alkoxycarbonyl, (C₁-C₈)-Alkylcarbonyl, (C₁-C₈)-Alkylcarbonyloxy, (C₁-C₈)-Alkylamino, Di-(C₁-C₈)-alkylamino, (C₁-C₆)-Hydroxyalkyl, -O-[CH₂-]ₓC_{f}H_{(2f+1-g)}F_{g}, -OCF₂Cl, -OCF₂-CHFCl, Carbamoyl, N-(C₁-C₈)-Alkylcarbamoyl, N,N-Di-(C₁-C₈)-alkylcarbamoyl, (C₁-C₈)-Alkylcarbonyloxy, (C₃-C₈)-Cycloalkyl, Phenyl, Benzyl, Phenoxy, Benzyloxy, Aminoalkyl, N-(C₁-C₈)-alkylamino (C₁-C₁₂)-alkyl oder N,N-Di-(C₁-C₈)-alkylamino-(C₁-C₁₂)-alkyl, (C₁-C₈)-Alkoxycarbonyloxy, (C₁-C₈)-Alkoxy-(C₁-C₈)-alkoxycarbonyloxy, (C₆-C₁₂)-Aryloxycarbonyloxy, (C₇-C₁₁)-Aralkyloxycarbonyloxy, (C₇-C₁₁)-Aralkylcarbonyloxy, Cinnamoyl, Cinnamoyloxy, (C₆-C₁₂)-Arylcarbonyloxy, (C₃-C₈)-Alkenylcarbonyloxy, (C₃-C₈)-Alkinylcarbonyloxy, (C₃-C₈)-Cycloalkylcarbonyloxy, (C₁-C₁₂)-Alkoxy-(C₁-C₁₂)-alkoxy, (C₁-C₁₂)-Alkoxy-amino, (C₁-C₁₂)-Alkoxy-N(C₁-C₆)-alkylamino, (C₁-C₁₂)-Alkoxy-N,N (C₁-C₆)-dialkylamino, Carbamoyloxy, N-(C₁-C₈)-Alkylcarbamoyloxy, N,N-Di-(C₁-C₈)-alkylcarbamoyl, N-(C₃-C₈)-Cycloalkylcarbamoyl, N-(C₆-C₁₂)-Arylamino, N-(C₇-C₁₁)-Aralkylamino, N-Alkyl-Aralkylamino, N-Alkyl-Arylamino, (C₃-C₈)-Cycloalkanoylamino, (C₁-C₈)-Alkanoylamino, (C₆-C₁₂)-Aroylamino, (C₇-C₁₁)-Aralkanoylamino, (C₁-C₈)-Alkanoyl-(C₁-C₈)-alkylamino, (C₃-C₈)-Cycloalkanoyl-(C₁-C₈)-alkylamino, (C₆-C₁₂)-Aroyl-(C₁-C₈)-alkylamino, (C₇-C₁₁)-Aralkanoyl-(C₁-C₈)-alkylamino, (C₁-C₈)-Alkylmercapto, (C₁-C₈)-Alkylsulfinyl, (C₁-C₈)-Alkylsulfonyl, (C₁-C₈)-Alkylcarbonyl, (C₃-C₈)-Cycloalkylcarbonyl, Nitro, Trifluormethyl, Phenylmercapto, Phenylsulfonyl, Phenylsulfinyl, Sulfamoyl, N-(C₁-C₆)-Alkylsulfamoyl, N,N-Di-(C₁-C₆)-alkylsulfamoyl, (C₁-C₈)-Alkyl-sulfonamido, Arylsulfonamido,
C im Falle von X = -N(R⁷) einen unsubstituierten oder substituierten (C₁-C₁₂)Alkoxyrest, (C₃-C₈)-Cycloalkoxyrest, (C₆-C₁₂)-Aryloxyrest oder einen (C₇-C₁₁)-Aralkyloxyrest bedeuten, der einfach oder mehrfach, vorzugsweise ein- und zweifach substituiert ist mit
Halogen, Trifluormethyl, (C₁-C₆)-Alkoxy, Hydroxy, (C₁-C₆)-Hydroxyalkyl, NR'R'' oder Cyano,
wobei jeweils
R' und R'' gleich oder verschieden sind und Wasserstoff, (C₆-C₁₂)-Aryl, (C₁-C₈)-Alkyl, (C₁-C₈)-Alkylcarbonyl, (C₇-C₁₁)Aralkylcarbonyl oder (C₆-C₁₂)-Arylcarbonyl bedeuten oder mit dem Stickstoff einen gesättigten heterocyclischen Ring bilden, vorzugsweise einen 5- oder 6-gliedrigen Ring,
und
R², R⁵ und R⁴ oder R³, sofern R⁴ oder R³ nicht schon oben definiert ist, gleich oder verschieden sind und
D Wasserstoff, wobei mindestens ein Rest R², R⁵ und R⁴ oder R³ ungleich Wasserstoff ist, Halogen, insbesondere Fluor, Chlor oder Brom, Cyano, Nitro, Trifluormethyl, (C₁-C₁₂)-Alkyl, -O-[CH₂-]ₓC_{f}H_{(2f+1-g)}F_{g}, -OCF₂Cl, -O-CF₂-CHFCl, (C₁-C₈)-Alkylmercapto, (C₁-C₈)-Alkylsulfinyl, (C₁-C₈)-Alkylsulfonyl, (C₁-C₈)-Alkylcarbonyl, Carbamoyl, N-(C₁-C₄)-Alkylcarbamoyl, N,N-Di-(C₁-C₄)-alkylcarbamoyl, (C₃-C₈)-Cycloalkyl, Phenylmercapto, Phenylsulfonyl, Phenylsulfinyl, (C₁-C₁₂)-Alkoxycarbanoyl, (C₁-C₁₂)-Alkylcarbanoyloxy, Amino, N-(C₁-C₁₀)-Alkylamino, Di-N,N-(C₁-C₁₀)-alkylamino, N,N-(C₃-C₈)-Alkandiylamino, wie z. B. Pyrollidino, Piperidino oder ihre heterocyclischen Derivate Morpholino, Thiomorpholino, N-(C₆-C₁₂)-Arylamino, N-(C₆-C₁₂)-Aryl-N-(C₁-C₁₀)-alkylamino, N-(C₇-C₁₁)-Aralkylamino, N-(C₇-C₁₁)-Aralkyl-N-(C₁-C₁₀)-alkylamino, (C₁-C₁₂)-Alkanoylamino, (C₃-C₈)-Cycloalkanoylamino(C₁-C₁₂)-Hydroxyalkanoylamino, (C₁-C₈)-Alkoxy-(C₁-C₁₂)alkanoylamino, (C₆-C₁₂)-Arylcarbonylamino, (C₇-C₁₁)-Aralkylcarbonylamino, (C₁-C₈)-Alkoxycarbonyloxy, (C₁-C₈)-Alkoxy-(C₁-C₈)-alkoxycarbonyloxy, (C₆-C₁₂)-Aryloxycarbonyloxy, (C₇-C₁₁)-Aralkyloxy carbonyloxy, (C₇-C₁₁)-Aralkylcarbonyloxy, (C₆-C₁₂)-Arylcarbonyloxy, (C₃-C₈)-Alkenylcarbonyloxy, (C₃-C₈)-Alkinylcarbonyloxy, (C₃-C₈)-Cycloalkylcarbonyloxy, (C₁-C₁₂)-Alkoxy-(C₁-C₁₂)-alkoxy, (C₁-C₁₂)-Alkoxyamino, (C₁-C₁₂)-Alkoxy-N-(C₁-C₆)-alkylamino, (C₁-C₁₂)-Alkoxy-N,N-(C₁-C₆)-dialkylamino, Carbamoyloxy, N-(C₁-C₈)-Alkylcarbamoyloxy, N,N-Di-(C₁-C₈)-alkylcarbamoyloxy, N-(C₃-C₈)-Cycloalkylcarbamoyloxy, NR'R'', (C₁-C₈)-Alkylmercapto, (C₁-C₈)-Alkylsulfinyl, (C₁-C₈)-Alkylsulfonyl, (C₁-C₈)-Alkylcarbonyl, (C₃-C₈)-Cycloalkylcarbonyl, Sulfamoyl, N-(C₁-C₆)-Alkylsulfamoyl, N,N-Di-(C₁-C₆)-alkylsulfamoyl, (C₁-C₈)-Alkylsulfonamido oder Arylsulfonamido bedeutet, wobei die in vorstehenden Substituenten vorhandenen Aryl- und Aralkylreste auch heterocyclischer Natur sein können und/oder, wie auch Alkyl, mit 1, 2, 3, 4 oder 5 gleichen oder verschiedenen Substituenten aus der Reihe Halogen, Cyano, Nitro, Trifluormethyl, (C₁-C₆)-Alkyl, Hydroxy, (C₁-C₆)-Hydroxyalkyl, (C₁-C₆)-Alkoxy, -O-[CH₂-]ₓC_{f}H_{(2f+1-g)}F_{g}, -OCF₂Cl, -O-CF₂-CHFCl, Trifluormethyl, (C₁-C₆)-Alkylmercapto, (C₁-C₆)-Alkylsulfinyl, (C₁-C₆)-Alkylsulfonyl, (C₁-C₆)-Alkylcarbonyl, (C₁-C₆)-Alkoxycarbonyl, Carbamoyl, N-(C₁-C₄)-Alkylcarbamoyl, N,N-Di-(C₁-C₄)-alkylcarbamoyl, (C₁-C₆)-Alkylcarbonyloxy, (C₃-C₈)-Cycloalkyl, Phenyl, Benzyl, Phenoxy, Benzyloxy, NR'-R'', Phenylmercapto, Phenylsulfonyl, Phenylsulfinyl, Sulfamoyl, N-(C₁-C₄)-Alkylsulfamoyl oder N,N-Di-(C₁-C₄)-alkylsulfamoyl, insbesondere mit bis zu 3 der vorstehend genannten gleichen oder verschiedenen Substituenten substituiert sind und eine CH₂-Gruppe der Alkylkette gegebenenfalls durch O, S, SO, SO₂ oder NR' ersetzt ist,
oder
E einen Alkyl-, Alkenyl- oder Alkinylrest mit bis zu 9 C-Atomen bedeutet, welcher gegebenenfalls substituiert ist mit
1, 2, 3, 4 oder 5 gleichen oder verschiedenen Substituenten aus der Reihe Hydroxy, Halogen, Cyano, Nitro, Trifluormethyl, (C₁-C₆)-Hydroxyalkyl, (C₁-C₆)Alkoxy, [CH₂-]ₓC_{f}H_{(2f+1-g)}F_{g}, -OCF₂-CHFCl, (C₁-C₆)-Alkylmercapto, (C₁-C₆)-Alkylsulfinyl, (C₁-C₆)-Alkylsulfonyl, (C₁-C₆)-Alkylcarbonyl, (C₁-C₆)-Alkoxycarbonyl, Carbamoyl, N-(C₁-C₄)-Alkylcarbamoyl, N,N-Di-(C₁-C₄)-alkylcarbamoyl, (C₁-C₆)-Alkylcarbonyloxy, (C₃-C₈)-Cycloalkyl, Carboxyl, Phenyl, Benzyl, Phenoxy, Benzyloxy, Phenylmercapto, Phenylsulfonyl, Phenylsulfinyl, Sulfamoyl, N-(C₁-C₄)-Alkylsulfamoyl, N,N-Di-(C₁-C₄)-alkylsulfamoyl, Amino, N-(C₁-C₁₀)-Alkylamino, Di-N,N-(C₁-C₁₀)-alkylamino, N,N-(C₃-C₈)-Alkandiylamino, wie z. B. Pyrollidino, Piperidino oder ihre heterocyclischen Derivate Morpholino, Thiomorpholino, N-(C₆-C₁₂)-Arylamino, N-(C₆-C₁₂)-Aryl-N-(C₁-C₁₀)-alkylamino, N-(C₇-C₁₁)-Aralkylamino, N-(C₇-C₁₁)-Aralkyl-N-(C₁-C₁₁)-alkylamino, (C₁-C₁₂)-Alkanoylamino, (C₃-C₈)-Cyclo-alkanonylamino, (C₁-C₁₂)-Hydroxyalkanoylamino, (C₁-C₈)-Alkoxy-(C₁-C₁₂)-alkanoylamino, (C₆-C₁₂)-Arylcarbonylamino, (C₇-C₁₁)-Aralkylocarbonylamino, insbesondere mit bis zu 3 der vorstehend genannten gleichen oder verschiedenen Substituenten und eine CH₂-Gruppe der Alkylkette gegebenenfalls durch O, S, SO, SO₂ oder NR' ersetzt ist, oder mit einem unsubstituierten oder substituierten (C₆-C₁₂)-Arylrest, (C₇-C₁₁)-Aralkylrest oder Heteroarylrest, der 1, 2, 3, 4 oder 5 gleiche oder verschiedene Substituenten aus der Reihe Hydroxy, Halogen, Cyano, Nitro, Trifluormethyl, (C₁-C₆)-Hydroxyalkyl, (C₁-C₆)-Alkyl, (C₁-C₆)Alkoxy, [CH₂-]ₓC_{f}H_{(2f+1-g)}F_{g'} -OCF₂-CHFCl, (C₁-C₆)-Alkylmercapto, (C₁-C₆)-Alkylsulfinyl, (C₁-C₆)-Alkylsulfonyl, (C₁-C₆)-Alkylcarbonyl, (C₁-C₆)-Alkoxycarbonyl, Carbamoyl, N-(C₁-C₄)-Alkylcarbamoyl, N,N-Di-(C₁-C₄)-alkylcarbamoyl, (C₁-C₆)-Alkylcarbonyloxy, (C₃-C₈)-Cycloalkyl, Carboxyl, Phenyl, Benzyl, Phenoxy, Benzyloxy, Phenylmercapto, Phenylsulfonyl, Phenylsulfinyl, Sulfamoyl, N-(C₁-C₄)-Alkylsulfamoyl, Amino, N-(C₁-C₁₀)-Alkylamino, Di-N,N-(C₁-C₁₀)-alkylamino, N,N-(C₃-C₈)-Alkandiylamino, wie z. B. Pyrollidino, Piperidino oder ihre heterocyclischen Derivate Morpholino, Thiomorpholino, N-(C₆-C₁₂)-Arylamino, N-(C₆-C₁₂)-Aryl-N-(C₁-C₁₀)-alkylamino, N-(C₇-C₁₁)aralkylamino, N-(C₇-C₁₁)-Aralkyl-N-(C₁-C₁₀)alkylamino, (C₁-C₁₂)-Alkanoylamino, (C₃-C₈)-Cyclo-alkanonylamino, (C₁-C₁₂)-Hydroxyalkanoylamino, (C₁-C₈)-Alkoxy-(C₁-C₁₂)-alkanoylamino, (C₆-C₁₂)-Arylcarbonylamino, (C₇-C₁₁)-Aralkylcarbonylamino, insbesondere mit bis zu 3 der vorstehend genannten gleichen oder verschiedenen Substituenten und eine CH₂-Gruppe der Alkylkette gegebenenfalls durch O, S, SO, SO₂ oder NR' ersetzt ist, oder
F einen substituierten oder unsubstituierten (C₆-C₁₂)-Arylrest, (C₇-C₁₁)-Aralkylrest oder Heteroarylrest, wobei der genannte Aryl- und Heteroarylrest, insbesondere Phenyl, Naphthyl, Thienyl, Furyl, Pyrrolyl oder Pyridin bedeutet, und der weiterhin
1,2,3,4 oder 5 gleiche oder verschiedene Substituenten aus der Reihe Hydroxy, Halogen, Cyano, Nitro, (C₁-C₆)-Alkyl, (C₁-C₆)-Alkoxy, Carboxyl, Trifluormethyl, (C₁-C₆)-Hydroxyalkyl, -O-[CH₂-]ₓC_{f}H_{(2f+1g)}F_{g'} -OCF₂Cl, -OCF₂-CHFCl, (C₁-C₆)-Alkylmercapto, (C₁-C₆)-Alkylsulfonyl, (C₁-C₆)-Alkylsulfinyl, (C₁-C₆)Alkylcarbonyl, (C₁-C₆)-Alkoxycarbonyl, Carbamoyl, N-(C₁-C₄)-Alkylcarbamoyl, N,N-Di-(C₁-C₄)-alkylcarbamoyl, (C₁-C₆)-Alkylcarbonyloxy, (C₃-C₈)-Cycloalkyl, Phenyl, Benzyl, Phenoxy, Benzyloxy, Amino, N-(C₁-C₁₀)-Alkylamino, Di-N,N-(C₁-C₁₀)-alkylamino, N,N-(C₃-C₈)-Alkandiylamino, wie z. B. Pyrollidino, Piperidino, Morpholino, Thiomorpholino, (C₁-C₁₀)-Alkanoylamino, (C₆-C₁₂)-Arylcarbonylamino, (C₇-C₁₁)-Aralkylcarbonylamino, Phenylmercapto, Phenylsulfonyl, Phenylsulfinyl, Sulfamoyl, N-(C₁-C₄)-Alkylsulfamoyl oder N,N-Di-(C₁-C₄)-alkylsulfamoyl, insbesondere bis zu 3 der vorstehend genannten gleichen oder verschiedenen Substituenten im Arylteil trägt, wobei eine CH₂-Gruppe der Arylkette gegebenenfalls durch O, S, SO, SO₂ oder NR' ersetzt ist, oder
G einen Substituenten der Formeln -OR⁹ oder -N(R⁹)₂ ist, wobei
R⁹ Wasserstoff, Alkyl, Alkenyl oder Alkinyl jeweils mit bis zu 9 C-Atomen, einen (C₆-C₁₂)-Arylrest oder einen Heteroarylrest bedeutet, der
1,2,3,4 oder 5 gleiche oder verschiedene Substituenten aus der Reihe Hydroxy, Halogen, Hydroxy, Cyano, Nitro, Carboxyl, (C₁-C₆)-Alkyl, (C₁-C₆)-Alkoxy, Trifluormethyl, (C₁-C₆)-Hydroxyalkyl, -O-[CH₂-]ₓC_{f}H_{(2f+1g)}F_{g}, -OCF₂Cl, -OCF₂-CHFCl, (C₁-C₆)-Alkylmercapto, (C₁-C₆)Alkylsulfonyl, (C₁-C₆)-Alkylsulfinyl, (C₁-C₆)-Alkylcarbonyl, (C₁-C₆)-Alkoxycarbonyl, Carbamoyl, N-(C₁-C₄)-Alkylcarbamoyl, N,N-Di-(C₁-C₄)-alkylcarbamoyl, (C₁-C₆)-Alkylcarbonyloxy, (C₃-C₈)-Cycloalkyl, Phenyl, Benzyl, Phenoxy, Bezyloxy, Amino, N-(C₁-C₁₀)-Alkylamino, Di-N,N-(C₁-C₁₀)-alkylamino, N,N-(C₃-C₈)-Alkandiylamino, wie z. B. Pyrollidino, Piperidino, Morpholino, Thiomorpholino, (C₁-C₁₀)-Alkanoylamino, (C₆-C₁₂)-Arylcarbonylamino, (C₇-C₁₁)-Aralkylcarbonylamino, Phenylmercapto, Phenylsulfonyl, Phenylsulfinyl, Sulfamoyl, N-(C₁-C₄)-Alkylsulfamoyl oder N,N-Di-(C₁-C₄)-alkylsulfamoyl, insbesondere bis zu 3 der vorstehend genannten gleichen oder verschiedenen Substituenten im Arylteil trägt, wobei eine CH₂-Gruppe der Arylkette gegebenenfalls durch O, S, SO, SO₂ oder NR' ersetzt ist,
und
n = O der 1,
f = 1 bis 8, vorzugsweise 1 bis 5,
g = 0,1 bis (2f+1),
x 0, 1, 2 oder 3, vorzugsweise 0 oder 1 ist,
zuzüglich alle Derivate, die in ihren Amino- oder Hydroxygruppen eine entsprechende Schutzgruppe aufweisen, sowie die physiologisch wirksamen Salze.

2. Verbindungen gemäß Anspruch 1, dadurch gekennzeichnet, daß
R¹ und R³ oder R⁴ -C(O)-X-R⁶ bedeutet, wobei
X -N(R⁷)- ist.

3. Verbindungen gemäß Ansprüche 1 oder 2, dadurch gekennzeichnet, daß
R⁶ Wasserstoff oder Methyl ist und
R⁷ die in Anspruch 1 angegebene Bedeutung hat oder
R⁶ und R⁷ Wasserstoff und/oder Methyl ist, sofern zumindest eine Gruppe R¹, R³ oder R⁴ einen Rest -C(O)-N(R⁷)-R⁶ bedeutet, worin R⁶ und/oder R⁷ die in Anspruch 1 angegebene Bedeutung hat.

4. Verbindungen gemäß den Ansprüchen 1 oder 2, dadurch gekennzeichnet, daß
A einen verzweigten oder unverzweigten (C₁-C₁₂)-Alkylrest bedeutet, der unsubstituiert ist oder einfach oder mehrfach substituiert ist mit
Halogen, insbesondere Fluor, Chlor, Brom, Hydroxy, Cyano, Carboxyl, (C₁-C₄)-Alkoxy, (C₁-C₄)-Alkoxycarboxyl, (C₁-C₈)-Alkoxycarbonyloxy, (C₁-C₈)-Alkoxy-(C₁-C₈)-alkoxycarbonyloxy, (C₆-C₁₂)-Aryloxycarbonyloxy, (C₇-C₁₁)-Aralkyloxycarbonyloxy, (C₇-C₁₁)-Aralkylcarbonyloxy, (C₇-C₁₁)-Arylcarbonyloxy, (C₃-C₈)-Cycloalkylcarbonyloxy, (C₁-C₁₂)-Alkoxy-(C₁-C₁₂)-alkoxy, Carbamoyloxy, N-(C₁-C₈)-Alkylcarbamoyloxy, N,N-Di (C₁-C₈)-alkylcarbamoyl, N-(C₃-C₈)-Cycloalkylcarbamoyl, N-(C₇-C₁₁)-Aralkylcarbamoyloxy,
N (C₆-C₁₂)-Arylcarbamoyloxy, wobei die in vorstehenden Substituenten vorhandenen Aryl- und Aralkylreste auch heterocyclischer Natur sein können und/oder wie auch Alkyl mit 1 oder 2 gleichen oder verschiedenen Substituenten aus der Reihe Halogen, Trifluormethyl, Hydroxy, (C₁-C₃)-Alkyl, (C₁-C₃)-Hydroxyalkyl, (C₁-C₉)-Alkoxy, -O-[CH₂-]ₓC_{f}H_{(2f+1-g)}F_{g}, -OCF₂Cl, -O-CF₂-CHFCl, -(C₁-C₃)-Alkoxycarbonyl, Carbamoyl, (C₁-C₆)-Alkylcarbonyloxy, (C₃-C₈)-Cycloalkyl, Phenyl, Benzyl, Phenoxy, Benzyloxy, substituiert sind, oder mit
einem unsubstituierten oder substituierten (C₆-C₁₂)-Arylrest oder Heteroarylrest, der 1 oder 2 gleiche oder verschiedene Substituenten aus der Reihe Halogen, Nitro, Cyano, Carboxyl, Hydroxy, Trifluormethyl, (C₁-C₃)-Hydroxyalkyl, (C₁-C₃)-Alkoxycarbonyl, Carbamoyl, NR'R'', N-(C₁-C₄)-Alkylcarbamoyl, N,N-Di-(C₁-C₄)-alkylcarbamoyl, (C ₁-C₈)-Alkoxy-(C₁-C₈)-Alkyl, (C₁-C₃)-Alkylcarbonyloxy, Aminoalkyl oder N-(C₁-C₄)-alkylamino-(C₁-C₆)-alkyl trägt, wobei R' und R'' gleich oder verschieden sind und Wasserstoff, (C₆-C₁₂)-Aryl, oder (C₁-C₄)-Alkyl bedeuten, oder mit
einem unsubstituierten oder substituierten (C₆-C₁₀)-Aryloxyrest oder (C₇-C₁₁)-Aralkyloxyrest, der 1 oder 2 gleiche oder verschiedene Substituenten aus der Reihe Hydroxy, Halogen, Trifluormethyl, (C₁-C₃)-Alkyl, (C₁-C₃)-Hydroxyalkyl, (C₁-C₃)-Alkoxy, (C₁-C₃)-Alkylmercapto, (C₁-C₃)-Alkylsulfinyl, (C₁-C₃)-Alkylsulfonyl, (C₁-C₃)-Alkylcarbonyl, (C₁-C₃)-Alkoxycarbonyl, Carbamoyl, N-(C₁-C₄)-Alkylcarbamoyl, N,N-Di-(C₁-C₄)-alkylcarbamoyl, (C₁-C₃)-Alkylcarbonyloxy, NR'R'' trägt, wobei R' und R'' gleich oder verschieden sind und Wasserstoff, (C₆-C₁₀)-Aryl oder (C₁-C₄)-Alkyl bedeuten, oder mit
einem Rest der allgemeinen Formel II
- O - R⁸ (II)
worin
R⁸ eine über ihren Acylrest gebundene Aminosäure oder ein Derivat hiervon bedeutet,
B einen (C₆-C₁₂)Aryl- oder (C₇-C₁₁)Aralkylrest, vorzugsweise Phenyl, Benzyl und Phenethyl, bedeuten, welche unsubstituiert oder einfach substituiert sind mit Halogen, Cyano, Carboxyl, Hydroxy, (C₁-C₈)-Alkyl, (C₁-C₈)-Alkoxy, (C₁-C₈)-Alkylcarbonyl, (C₁-C₄)-Alkylcarbonyloxy, (C₁-C₄)-Alkoxycarbonyl, (C₁-C₄)-Hydroxyalkyl, Amino,
(C₁-C₅)-Alkylamino, Di-(C₁-C₅)alkylamino, (C₁-C₅)-Alkanoylamino, Carbamoyl, N-(C₁-C₄)-Alkylcarbamoyl, N,N-Di-(C₁-C₄)-alkylcarbamoyl, Carbamoyl, N-(C₁-C₄)-Alkylcarbamoyloxy, N,N-Di-(C₁-C₄)-alkylcarbamoyloxy, oder
C einen unsubstituierten (C₁-C₆)-Alkoxyrest, (C₃-C₈)-Cycloalkoxyrest, (C₆-C₁₂)-Aryloxyrest oder
(C₇-C₁₁)-Aralkyloxyrest bedeuten.

5. Verbindungen nach den Ansprüchen 1 bis 4, dadurch gekennzeichnet, daß R², R⁵ und R⁴ oder R³, sofern R⁴ oder R³ nicht schon in den Ansprüchen 1 bis 4 definiert sind, und nicht Wasserstoff sein sollen, stehen.
D Halogen, insbesondere Fluor, Chlor oder Brom, Cyano, Nitro, Trifluormethyl, (C₁-C₁₂)-Alkyl, -O-[CH₂-]ₓC_{f}H_{(2f+g)}F_{g}, -OCF₂Cl, -O-CF₂-CHFCl, (C₁-C₈)-Alkylcarbonyl, Carbamoyl, N-(C₁-C₄)-Alkylcarbamoyl, N,N-Di-(C₁-C₄)-alkylcarbamoyl, (C₃-C₈)-Cycloalkyl, (C₁-C₁₂)-Alkoxycarbonyl, (C₁-C₁₂)-Alkylcarbonyloxy, Amino, N-(C₁-C₁₀)-Alkylamino, Di-N,N-(C₁-C₁₀)-alkylamino, N,N-(C₃-C₈)-Alkandiylamino, wie z. B. Pyrollidino, Piperidino oder ihre heterocyclischen Derivate Morpholino, Thiomorpholino, N-(C₆-C₁₂)-Arylamino N-(C₆-C₁₂)-Aryl-N-(C₁-C₈)-alkylamino, N-(C₇-C₁₁)-Aralkylamino, N-(C₇-C₁₁)-Aralkyl-N-(C₁-C₁₀)-alkylamino, (C₆-C₁₂)-Arylcarbonylamino, (C₇-C₁₁)-Aralkylcarbonylamino, (C₁-C₆)-Alkoxycarbonyloxy, (C₆-C₁₂)-Aryloxycarbonyloxy, (C₇-C₁₁)-Aralkylcarbonyloxy, (C₆-C₁₂)-Arylcarbonyloxy, (C₃-C₈)-Alkenylcarbonyloxy, (C₃-C₈)-Cycloalkylcarbonyloxy, (C₁-C₁₂)-Alkoxy-(C₁-C₁₂)-alkoxy, (C₁-C₁₂)-Alkoxy-amino, (C₁-C₁₂)-Alkoxy-N-(C₁-C₆)-alkylamino, (C₁-C₁₂)-Alkoxy-N,N-(C₁-C₆)-dialkylamino, Carbamoyloxy, N-(C₁-C₆)-Alkylcarbamoyloxy, N,N-Di-(C₁-C₈)-alkylcarbamoyloxy, N-(C₃-C₈)-Cycloalkylcarbamoyloxy, NR'R'', (C₁-C₈)-Alkylmercapto, (C₁-C₆)-Alkylsulfinyl, (C₁-C₈)-Alkylsulfonyl, (C₁-C₈)-Alkylcarbonyl, (C₃-C₈)-Cycloalkylcarbonyl, wobei die in vorstehenden Substituenten vorhandenen Aryl- und Aralkylreste auch heterocyclischer Natur sein können und/oder, wie auch Alkyl, mit 1,2 oder 3 gleichen oder verschiedenen Substituenten aus der Reihe Halogen, Trifluormethyl, (C₁-C₆)-Alkyl Hydroxy, (C₁-C₆)-Hydroxyalkyl, (C₁-C₆)-Alkoxy, -O-[CH₂-]ₓC_{f}H_{(2f+1-g)}F_{g}, (C₃-C₈)-Cycloalkyl, Phenyl, Benzyl, Phenoxy, Benzyloxy, NR'-R'', Phenylmercapto, Phenylsulfonyl, Phenylsulfinyl, Sulfamoyl, N-(C₁-C₄)-Alkylsulfamoyl oder N,N-Di-(C₁-C₄)-alkylsulfamoyl,
oder
E einen Alkyl- oder Alkenylrest mit bis zu 5 C-Atomen bedeutet, welcher gegebenenfalls substituiert ist mit 1,2 oder 3 gleichen oder verschiedenen Substituenten aus der Reihe Hydroxy, Halogen, Cyano, Nitro, Trifluormethyl (C₁-C₆)-Hydroxyalkyl, (C₁-C₆)Alkoxy, [CH₂]ₓC_{f}H_{(2f+1-g)}F_{g},-OCF₂-CHFCl, (C₁-C₆)-Alkylcarbonyl, (C₁-C₆)-Alkoxycarbonyl, Carbamoyl, N-(C₁-C₄)-Alkylcarbamoyl, N,N-Di-(C₁-C₄)-alkylcarbamoyl, (C₁-C₆)-Alkylcarbonyloxy, (C₃-C₈)-Cycloalkyl, Carboxyl, Phenyl, Benzyl, Phenoxy, Benzyloxy, Amino, N-(C₁-C₆)-Alkylamino, Di-N,N-(C₁-C₆)-alkylamino, N,N-(C₃-C₈)-Alkandiylamino, wie z. B. Pyrollidino, Piperidino, N-(C₆-C₁₂)-Arylamino, N-(C₆-C₁₂)-Aryl-N-(C₁-C₆)-alkylamino, N-(C₇-C₁₁)-Aralkylamino, N-(C₇-C₁₁)-Aralkyl-N-(C₁-C₁₀)-alkylamino, (C₁-C₆)-Alkanoylamino, (C₁-C₈)-Alkoxy-(C₁-C₆)-alkanoylamino, (C₆-C₁₂)-Arylcarbonylamino, (C₇-C₁₁)-Aralkylocarbonylamino, oder mit einem unsubstituierten oder substituierten (C₆-C₁₂)-Arylrest, (C₇-C₁₁)-Aralkylrest oder Heterarylrest, der 1,2 oder 3 gleiche oder verschiedene Sustituenten aus der Reihe Hydroxy, Halogen, Cyano, Nitro, Trifluormethyl, (C₁-C₆)-Hydroxyalkyl, (C₁-C₆)-Alkoxy, (C₁-C₆)-Alkoxy, [CH₂-]ₓC_{f}H_{(2f+1-g)}F_{g}, (C₁-C₆)-Alkylcarbonyl, (C₁-C₆)-Alkoxycarbonyl, Carbamoyl, N-(C₁-C₄)-Alkylcarbamoyl, N,N-Di-(C₁-C₄)-alkylcarbamoyl, (C₁-C₆)-Alkylcarbonyloxy, (C₃-C₈)-Cycloalkyl, Carboxyl, Phenyl, Benzyl, Phenoxy, Benzyloxy, Amino, N-(C₁-C₆)-Alkylamino, Di-N,N-(C₁-C₆)-alkylamino, N,N-(C₃-C₈)-Alkandiylamino, wie z. B. Pyrollidino, Piperidino, N-(C₆-C₁₂)-Arylamino, N-(C₆-C₁₂)-Aryl-N-(C₁-C₆)-alkylamino, N-(C₇-C₁₁)-Aralkylamino, N-(C₇-C₁₁)-Aralkyl-N-(C₁-C₁₀)-alkylamino, (C₁-C₆)-Alkanoylamino, (C₃-C₈)-Cycloalkanoylamino, (C₁-C₆)-Hydroxyalkanoylamino, (C₁-C₆)-Alkoxy-(C₁-C₆)-alkanoylamino, (C₆-C₁₂)-Arylcarbonylamino, (C₇-C₁₁)-Aralkylcarbonylamino,
oder
F einen unsubstituierten oder substituierten (C₆-C₁₂)-Arylrest, (C₇-C₁₁)Aralkylrest oder Heteroarylrest, wobei der genannte Aryl- und Heteroarylrest insbesondere Phenyl, Naphthyl, Thienyl, Furyl, Pyrrolyl oder Pyridin bedeutet, und der weiterhin 1,2 oder 3 gleiche oder verschiedene Substituenten aus der Reihe Hydroxy, Halogen, Cyano, Nitro, (C₁-C₆)-Alkyl, (C₁-C₆)-Alkoxy, Carboxyl, Trifluormethyl, (C₁-C₆)-Hydroxyalkyl, -O-[CH₂-]ₓC_{f}H_{(2f+1-g)}F_{g}, (C₁-C₆)-Alkylcarbonyl, (C₁-C₆-)-Alkoxycarbonyl, Carbamoyl, N-(C₁-C₄)-Alkylcarbamoyl, N,N-Di-(C₁-C₄)-alkylcarbamoyl, (C₁-C₆)-Akylcarbonyloxy, (C₃-C₈)-Cycloalkyl, Phenyl, Benzyl, Phenoxy, Benzyloxy, Amino, N-(C₁-C₁₀)-Alkylamino, Di-N,N-(C₁-C₁₀)-alkylamino, N,N-(C₃-C₈)-Alkandiylamino, wie z. B. Pyrollidino, Piperidino, (C₁-C₁₀)-Alkanoxylamino, (C₆-C₁₂)-Arylcarbonylamino, (C₇-C₁₁)-Aralkylcarbonylamino,
oder
G einen Substituenten der Formeln -OR⁹ oder -N(R⁹)₂ ist, wobei
R⁹ Wasserstoff, Alkyl oder Alkenyl jeweils mit bis zu 6 C-Atomen, einen (C₆-C₁₂)-Arylrest oder einen Heteroarylrest bedeutet, der 1, 2 oder 3 gleiche oder verschiedene Substituenten aus der Reihe Halogen, Hydroxy, Cyano, Nitro, Carboxyl, Trifluormethyl, (C₁-C₆)-Alkyl, (C₁-C₆)-Hydroxyalkyl, (C₁-C₆)-Alkoxy, -O-[CH₂-]ₓC_{f}H_{(2f+1-g)}F_{g}, (C₁-C₆)-Alkylcarbonyl, (C₁-C₆)-Alkoxycarbonyl, Carbamoyl, N-(C₁-C₄)-Alkylcarbamoyl, N,N-Di-(C₁-C₄)-alkylcarbamoyl, (C₁-C₆)-Alkylcarbonyloxy, (C₃-C₈)-Cycloalkyl, Phenyl, Benzyl, Phenoxy, Benzyloxy, Amino, N-(C₁-C₁₀)-Alkylamino, Di-N,N-(C₁-C₁₀)-alkylamino, N,N-(C₃-C₈)-Alkandiylamino, wie z. B. Pyrollidino, Piperidino, (C₁-C₁₀)-Alkanoylamino, (C₆-C₁₂)-Arylcarbonylamino, (C₇-C₁₁)-Aralkylcarbonylamino.

6. Verbindungen nach den Ansprüchen 1 bis 5, dadurch gekennzeichnet, daß
R², R⁵ und R⁴ oder R³, sofern R⁴ oder R³ nicht schon in den Ansprüchen 1 bis 4 definiert sind und nicht Wasserstoff sein sollen, stehen für
D Halogen, insbesondere Fluor, Chlor oder Brom, Trifluormethyl, (C₁-C₁₂)-Alkyl, -O-[CH₂-]ₓC_{f}H_{(2f+1-g)}F_{g}, -OCF₂Cl, -O-CF₂-CHFCl, (C₁-C₈)-Alkylcarbonyl, (C₃-C₈)-Cycloalkyl, Amino, N-(C₁-C₁₀)-Alkylamino, Di-N,N-(C₁-C₁₀)-Alkylamino, N-(C₆-C₁₂)-Arylamino, N-(C₆-C₁₂)-Aryl-N-(C₁-C₈)-alkylamino, N-(C₇-C₁₁)-Aralkylamino, N-(C₇-C₁₁)-Aralkyl-N-(C₁-C₁₀)-alkylamino bedeutet, wobei die in vorstehenden Substituenten vorhandenen Aryl- und Aralkylreste auch heterocyclischer Natur sein können und/oder, wie auch Alkyl, mit 1 oder 2 gleichen oder verschiedenen Substituenten aus der Reihe Halogen, Trifluormethyl, (C₁-C₆)-Alkyl Hydroxy, (C₁-C₆)-Hydroxyalkyl, (C₁-C₆)-Alkoxy, -O-[CH₂-]ₓC_{f}H_{(2f+1-g)}F_{g}, Trifluormethyl, NR'-R substituiert sein können oder
E einen Alkyl- oder Alkenylrest mit bis zu 5 C-Atomen bedeutet, welcher gegebenenfalls substituiert ist mit 1 oder 2 gleichen oder verschiedenen Substituenten aus der Reihe Hydroxy, Halogen, Trifluormethyl, (C₁-C₆)-Hydroxyalkyl, (C₁-C₆)Alkoxy, (C₁-C₆)-Alkylcarbonyl, (C₁-C₆)-Alkoxycarbonyl, (C₁-C₆)-Alkylcarbonyloxy, Phenyl, Benzyl, Phenoxy, Benzyloxy, Amino, N-(C₁-C₆)-Alkylamino, Di-N,N-(C₁-C₆)-alkylamino oder mit
einem unsubstituierten oder substituierten (C₆-C₁₂)-Arylrest, (C₇-C₁₁)-Aralkylrest oder Heteroarylrest, der 1 oder 2 gleiche oder verschiedene Substituenten aus der Reihe Hydroxy, Halogen, Trifluormethyl, (C₁-C₆)-Alkyl, (C₁-C₆)-Hydroxyalkyl, (C₁-C₆)-Alkoxy, [CH₂-]ₓC_{f}H_{(2f+1-g)}F_{g}, (C₁-C₆)-Alkylcarbonyl, (C₁-C₆)-Alkoxycarbonyl, Phenyl, Benzyl, Phenoxy, Benzyloxy, Amino, N-(C₁-C₆)Alkylamino, Di-N,N-(C₁-C₆)-alkylamino, N,N-(C₃-C₈)-Alkandiylamino, wie z. B. Pyrollidino, Piperidino, oder
F einen unsubstituierten oder substituierten (C₆-C₁₂)-Arylrest, (C₇-C₁₁)Aralkylrest oder Heteroarylrest, wobei der genannte Aryl- und Heteroarylrest insbesondere Phenyl, Naphthyl, Thienyl, Furyl, Pyrrolyl oder Pyridin bedeutet, und der weiterhin 1,2 oder 3 gleiche oder verschiedene Substituenten aus der Reihe Hydroxy, Halogen, (C₁-C₆)-Alkyl, (C₁-C₆)-Alkoxy, Carboxyl, Trifluormethyl, (C₁-C₆)-Hydroxyalkyl, -O-[CH₂-]ₓC_{f}H_{(2f+1-g)}F_{g}, (C₁-C₆)-Alkylcarbonyl, (C₁-C₆)-Alkoxycarbonyl, Amino, N-(C₁-C₁₀)-Alkylamino, Di-N,N-(C₁-C₁₀)-alkylamino, oder
G einen Substituenten der Formeln -OR⁹ oder -N(R⁹)₂ ist, wobei
R⁹ Wasserstoff oder Alkyl jeweils mit bis zu 6 C-Atomen, einen (C₆-C₁₂)-Arylrest oder einen Heteroarylrest bedeutet, der 1 oder 2 gleiche oder verschiedene Substituenten aus der Reihe Halogen, Trifluormethyl, (C₁-C₆)-Alkyl, (C₁-C₆)-Alkoxy, - O-[CH₂-]ₓC_{f}H_{(2f+1-g)})F_{g}, Amino, N-(C₁-C₁₀)-Alkylamino oder Di-N,N-(C₁-C₁₀)-alkylamino enthält.

7. Verbindungen nach den Ansprüchen 1 bis 6, dadurch gekennzeichnet, daß
R², R⁵ und R⁴ oder R³, sofern R⁴ oder R³ nicht schon in den Ansprüchen 1 bis 4 definiert sind und nicht Wasserstoff sein sollen, stehen für
D Halogen, insbesondere Fluor, Chlor oder Brom, Trifluormethyl, (C₁-C₁₂)-Alkyl, -O-[CH₂-]ₓC_{f}H_{(2f+1-g)}F_{g}, -OCF₂Cl, -O-CF₂-CHFCl, (C₁-C₈)-Alkylcarbonyl, (C₃-C₈)-Cycloalkyl, Amino, N-(C₁-C₁₀)-Alkylamino, Di-N,N-(C₁-C₁₀)-Alkylamino, N-(C₆-C₁₂)-Arylamino, N-(C₆-C₁₂)-Aryl-N-(C₁-C₈)-alkylamino, N-(C₇-C₁₁)-Aralkylamino, N-(C₇-C₁₁)-Aralkyl-N-(C₁-C₁₀)-alkylamino bedeutet, oder:
E einen Alkyl- oder Alkenylrest mit bis zu 5 C-Atomen bedeutet, welcher gegebenenfalls substitutiert ist mit 1 oder 2 gleichen oder verschiedenen Substituenten aus der Reihe Hydroxy, Halogen, Trifluormethyl, (C₁-C₆)Alkoxy, (C₁-C₆)-Alkylcarbonyl, (C₁-C₆)-Alkoxycarbonyl, (C1-C₆)-Alkylcarbonyloxy, Phenyl, Phenoxy, Benzyloxy, Amino, N-(C₁-C₆)-Alkylamino, Di-N,N-(C₁-C₆)-alkylamino oder mit einem unsubstituierten oder substituierten (C₆-C₁₂)-Arylrest, (C₇-C₁₁)-Aralkylrest oder Heteroarylrest, der 1 oder 2 gleiche oder verschiedene Substituenten aus der Reihe Hydroxy, Halogen, Trifluormethyl, (C₁-C₆)-Alkyl, (C₁-C₆)-Hydroxyalkyl, (C₁-C₆)-Alkoxy, (C₁-C₆)-Alkylcarbonyl, (C₁-C₆)-Alkoxycarbonyl, oder
F einen unsubstituierten oder substituierten (C₆-C₁₂)-Arylrest, (C₇-C₁₁)Aralkylrest, wobei der genannte Arylrest insbesondere Phenyl oder Naphthyl, bedeutet und der weiterhin 1 oder 2 gleiche oder verschiedene Substituenten aus der Reihe Hydroxy, Halogen, (C₁-C₆)-Alkyl, (C₁-C₆)-Alkoxy, Carboxyl, Trifluormethyl, (C₁-C₆)-Hydroxyalkyl, (C₁-C₆)-Alkylcarbonyl, (C₁-C₆)-Alkoxycarbonyl, oder
G einen Substituenten der Formeln -OR⁹ oder -N(R⁹)₂ ist, wobei
R⁹ Wasserstoff oder Alkyl jeweils mit bis zu 6 C-Atomen, einen (C₆-C₁₂)-Arylrest oder einen Heteroarylrest bedeutet, der 1 oder 2 gleiche oder verschiedene Substituenten aus der Reihe Halogen, Trifluormethyl, (C₁-C₆)-Alkyl, (C₁-C₆)-Alkoxy, Amino, N-(C₁-C₁₀)-Alkylamino oder Di-N,N-(C₁-C₁₀)-alkylamino enthält.

8. Verfahren zur Herstellung von Verbindungen gemäß den Ansprüchen 1 bis 7, dadurch gekennzeichnet, daß Verbindungen der allgemeinen Formel V oder VI mit einem Oxidationsmittel in einem organischen Lösungsmittel bei einer Temperatur zwischen -30 und +40° C zu Verbindungen der allgemeinen Formel I umgesetzt werden.

9. Verbindungen nach den Ansprüchen 1 bis 7 zur Inhibierung der Prolin- und Lysinhydroxylase.

10. Verbindungen nach den Ansprüchen 1 bis 7 zur Anwendung als Fibrosuppresiva und Immunsuppresiva.

11. Arzneimittel, enthaltend eine Verbindung der Formel I und einen verträglichen pharmazeutischen Träger.

12. Verwendung von Verbindungen der Formel I zur Beeinflussung des Stoffwechsels von Collagen und collagenähnlichen Stoffen sowie der Biosynthese von C1_{q}.

13. Verwendung von Verbindungen der Formel I zur Behandlung von Störungen des Stoffwechsels von Collagen und collagenähnlichen Stoffen sowie der Biosynthese von C1_{q}.

14. Verfahren zur Herstellung von Arzneimitteln zur Beeinflussung des Stoffwechsels von Collagen und collagenähnlichen Stoffen sowie der Biosynthese von C1_{q}, dadurch gekennzeichnet, daß das Arzneimittel eine Verbindung der Formel I enthält.
